# EUROPEAN PATENT SPECIFICATION

(11) **EP 1 100 928 B1**
(45) Date of publication and mention of the grant of the patent: **01.02.2006**
(21) Application number: 99937729.4
(22) Date of filing: 30.07.1999
(51) Int. Cl.: C12N 15/53, C12N 15/54, C12N 9/10, C12N 9/02, C12N 15/62, C12N 15/82

(54) **BIFUNCTIONAL ENZYMES FOR BIOPOLYMER PRODUCTION**
BIFUNKTIONELLE ENZYME ZUR BIOPOLYMERHERSTELLUNG
ENZYMES BIFONCTIONNELS POUR LA PRODUCTION DE BIOPOLYMERES

(30) Priority: 30.07.1998 US 94674 P
(43) Date of publication of application: 23.05.2001
(73) Proprietor: METABOLIX, INC., Cambridge, MA 02139 (US)
(72) Inventor: PEOPLES, Oliver, P., Arlington, MA 02474 (US); MADISON, Lara, Bridgewater, MA 02324 (US); HUISMAN, Gjalt, W., San Carlos, CA 94070-3419 (US)
(74) Representative: Bassett, Richard Simon
(86) International application number: PCT/US1999/017452
(87) International publication number: WO 2000/006747

(56) References cited:
- WO-A-91/00917
- WO-A-95/05472
- WO-A-98/06854
- FUKUI ET AL: "Cloning and analysis of the poly(3- hydroxybutyrate -co-3-hydroxyhexanoate) biosynthesis genes of Aeromonas caviae" JOURNAL OF BACTERIOLOGY,US,WASHINGTON, DC, vol. 179, no. 15, August 1997 (1997-08), pages 4821-4830, XP002113257 ISSN: 0021-9193

### SEQUENCE LISTING

<110> Metabolix, Inc.
<120> Enzymes for Biopolymer Production
<130> MBX 030 PCT
<140> PCT/US99/17452
   <141> 1999-07-30
<150> 60/094,674
   <151> 1998-07-30
<160> 61
<170> PatentIn Ver. 2.1
<210> 1
   <211> 1182
   <212> DNA
   <213> Alcaligenes eutrophus
<220>
   <221> gene
   <222> (1) .. (1182)
   <223> phbA gene
<400> 1
   atgactgacg ttgtcatcgt atccgccgcc cgcaccgcgg tcggcaagtt tggcggctcg 60
   ctggccaaga tcccggcacc ggaactgggt gccgtggtca tcaaggccgc gctggagcgc 120
   gccggcgtca agccggagca ggtgagcgaa gtcatcatgg gccaggtgct gaccgccggt 180
   tcgggccaga accccgcacg ccaggccgcg atcaaggccg gcctgccggc gatggtgccg 240
   gccatgacca tcaacaaggt gtgcggctcg ggcctgaagg ccgtgatgct ggccgccaac 300
   gcgatcatgg cgggcgacgc cgagatcgtg gtggccggcg gccaggaaaa catgagcgcc 360
   gccccgcacg tgctgccggg ctcgcgcgat ggtttccgca tgggcgatgc caagctggtc 420
   gacaccatga tcgtcgacgg cctgtgggac gtgtacaacc agtaccacat gggcatcacc 480
   gccgagaacg tggccaagga atacggcatc acacgcgagg cgcaggatga gttcgccgtc 540
   ggctcgcaga acaaggccga agccgcgcag aaggccggca agtttgacga agagatcgtc 600
   ccggtgctga tcccgcagcg caagggcgac ccggtggcct tcaagaccga cgagttcgtg 660
   cgccagggcg ccacgctgga cagcatgtcc ggcctcaagc ccgccttcga caaggccggc 720
   acggtgaccg cggccaacgc ctcgggcctg aacgacggcg ccgccgcggt ggtggtgatg 780
   tcggcggcca aggccaagga actgggcctg accccgctgg ccacgatcaa gagctatgcc 840
   aacgccggtg tcgatcccaa ggtgatgggc atgggcccgg tgccggcctc caagcgcgcc 900
   ctgtcgcgcg ccgagtggac cccgcaagac ctggacctga tggagatcaa cgaggccttt 960
   gccgcgcagg cgctggcggt gcaccagcag atgggctggg acacctccaa ggtcaatgtg 1020
   aacggcggcg ccatcgccat cggccacccg atcggcgcgt cgggctgccg tatcctggtg 1080
   acgctgctgc acgagatgaa gcgccgtgac gcgaagaagg gcctggcctc gctgtgcatc 1140
   ggcggcggca tgggcgtggc gctggcagtc gagcgcaaat aa                    1182
<210> 2
   <211> 393
   <212> PRT
   <213> Alcaligenes eutrophus
<220>
   <221> PEPTIDE
   <222> (1)..(393)
   <223> beta-ketothiolase
<400> 2
   Met Thr Asp Val Val Ile Val Ser Ala Ala Arg Thr Ala Val Gly Lys
   1               5                   10                  15
   Phe Gly Gly Ser Leu Ala Lys Ile Pro Ala Pro Glu Leu Gly Ala Val
               20                  25                  30
   Val Ile Lys Ala Ala Leu Glu Arg Ala Gly Val Lys Pro Glu Gln Val
           35                  40                  45
   Ser Glu Val Ile Met Gly Gln Val Leu Thr Ala Gly Ser Gly Gln Asn
       50                  55                  60
   Pro Ala Arg Gln Ala Ala Ile Lys Ala Gly Leu Pro Ala Met Val Pro
   65                  70                  75                  80
   Ala Met Thr Ile Asn Lys Val Cys Gly Ser Gly Leu Lys Ala Val Met
                   85                  90                  95
   Leu Ala Ala Asn Ala Ile Met Ala Gly Asp Ala Glu Ile Val Val Ala
               100                 105                 110
   Gly Gly Gln Glu Asn Met Ser Ala Ala Pro His Val Leu Pro Gly Ser
           115                 120                 125
   Arg Asp Gly Phe Arg Met Gly Asp Ala Lys Leu Val Asp Thr Met Ile
       130                 135                 140
   Val Asp Gly Leu Trp Asp Val Tyr Asn Gln Tyr His Met Gly Ile Thr
   145                 150                 155                 160
   Ala Glu Asn Val Ala Lys Glu Tyr Gly Ile Thr Arg Glu Ala Gln Asp
                   165                 170                 175
   Glu Phe Ala Val Gly Ser Gln Asn Lys Ala Glu Ala Ala Gln Lys Ala
   180 185 190
   Gly Lys Phe Asp Glu Glu Ile Val Pro Val Leu Ile Pro Gln Arg Lys
   195 200 205
   Gly Asp Pro Val Ala Phe Lys Thr Asp Glu Phe Val Arg Gln Gly Ala
   210 215 220
   Thr Leu Asp Ser Met Ser Gly Leu Lys Pro Ala Phe Asp Lys Ala Gly
   225 230 235 240
   Thr Val Thr Ala Ala Asn Ala Ser Gly Leu Asn Asp Gly Ala Ala Ala
   245 250 255
   Val Val Val Met Ser Ala Ala Lys Ala Lys Glu Leu Gly Leu Thr Pro
   260 265 270
   Leu Ala Thr Ile Lys Ser Tyr Ala Asn Ala Gly Val Asp Pro Lys Val
   275 280 285
   Met Gly Met Gly Pro Val Pro Ala Ser Lys Arg Ala Leu Ser Arg Ala
   290 295 300
   Glu Trp Thr Pro Gln Asp Leu Asp Leu Met Glu Ile Asn Glu Ala Phe
   305 310 315 320
   Ala Ala Gln Ala Leu Ala Val His Gln Gln Met Gly Trp Asp Thr Ser
   325 330 335
   Lys Val Asn Val Asn Gly Gly Ala Ile Ala Ile Gly His Pro Ile Gly
   340 345 350
   Ala Ser Gly Cys Arg Ile Leu Val Thr Leu Leu His Glu Met Lys Arg
   355 360 365
   Arg Asp Ala Lys Lys Gly Leu Ala Ser Leu Cys Ile Gly Gly Gly Met
   370 375 380
   Gly Val Ala Leu Ala Val Glu Arg Lys
   385 390
<210> 3
   <211> 43
   <212> DNA
   <213> Artificial Sequence
<220>
   <223> Description of Artificial Sequence: oligonucleotide primer- AlFKpn
<400> 3
   ggggtaccag gaggttttta tgactgacgt tgtcatcgta tcc 43
<210> 4
   <211> 37
   <212> DNA
   <213> Artificial Sequence
<220>
   <223> Description of Artificial Sequence: oligonucleotide primer- A1F-Bam
<400> 4
   cgcggatcct ttgcgctcga ctgccagcgc cacgccc 37
<210> 5
   <211> 741
   <212> DNA
   <213> Alcaligenes eutrophus
<220>
   <221> gene
   <222> (1) .. (741)
   <223> phbB gene
<400> 5
   atgactcagc gcattgcgta tgtgaccggc ggcatgggtg gtatcggaac cgccatttgc 60
   cagcggctgg ccaaggatgg ctttcgtgtg gtggccggtt gcggccccaa ctcgccgcgc 120
   cgcgaaaagt ggctggagca gcagaaggcc ctgggcttcg atttcattgc ctcggaaggc 180
   aatgtggctg actgggactc gaccaagacc gcattcgaca aggtcaagtc cgaggtcggc 240
   gaggttgatg tgctgatcaa caacgccggt atcacccgcg acgtggtgtt ccgcaagatg 300
   acccgcgccg actgggatgc ggtgatcgac accaacctga cctcgctgtt caacgtcacc 360
   aagcaggtga tcgacggcat ggccgaccgt ggctggggcc gcatcgtcaa catctcgtcg 420
   gtgaacgggc agaagggcca gttcggccag accaactact ccaccgccaa ggccggcctg 480
   catggcttca ccatggcact ggcgcaggaa gtggcgacca agggcgtgac cgtcaacacg 540
   gtctctccgg gctatatcgc caccgacatg gtcaaggcga tccgccagga cgtgctcgac 600
   aagatcgtcg cgacgatccc ggtcaagcgc ctgggcctgc cggaagagat cgcctcgatc 660
   tgcgcctggt tgtcgtcgga ggagtccggt ttctcgaccg gcgccgactt ctcgctcaac 720
   ggcggcctgc atatgggctg a 741
<210> 6
   <211> 246
   <212> PRT
   <213> Alcaligenes eutrophus
<220>
   <221> PEPTIDE
   <222> (1)..(246)
   <223> reductase
<400> 6
   Met Thr Gln Arg Ile Ala Tyr Val Thr Gly Gly Met Gly Gly Ile Gly
    1               5                  10                  15
   Thr Ala Ile Cys Gln Arg Leu Ala Lys Asp Gly Phe Arg Val Val Ala
               20                  25                  30
   Gly Cys Gly Pro Asn Ser Pro Arg Arg Glu Lys Trp Leu Glu Gln Gln
           35                  40                  45
   Lys Ala Leu Gly Phe Asp Phe Ile Ala Ser Glu Gly Asn Val Ala Asp
       50                  55                  60
   Trp Asp Ser Thr Lys Thr Ala Phe Asp Lys Val Lys Ser Glu Val Gly
   65                  70                  75                  80
   Glu Val Asp Val Leu Ile Asn Asn Ala Gly Ile Thr Arg Asp Val Val
                   85                  90                  95
   Phe Arg Lys Met Thr Arg Ala Asp Trp Asp Ala Val Ile Asp Thr Asn
               100                 105                 110
   Leu Thr Ser Leu Phe Asn Val Thr Lys Gln Val Ile Asp Gly Met Ala
           115                 120                 125
   Asp Arg Gly Trp Gly Arg Ile Val Asn Ile Ser Ser Val Asn Gly Gln
       130                 135                 140
   Lys Gly Gln Phe Gly Gln Thr Asn Tyr Ser Thr Ala Lys Ala Gly Leu
   145                 150                 155                 160
   His Gly Phe Thr Met Ala Leu Ala Gln Glu Val Ala Thr Lys Gly Val
                   165                 170                 175
   Thr Val Asn Thr Val Ser Pro Gly Tyr Ile Ala Thr Asp Met Val Lys
               180                 185                 190
   Ala Ile Arg Gln Asp Val Leu Asp Lys Ile Val Ala Thr Ile Pro Val
           195                 200                 205
   Lys Arg Leu Gly Leu Pro Glu Glu Ile Ala Ser Ile Cys Ala Trp Leu
       210                 215                 220
   Ser Ser Glu Glu Ser Gly Phe Ser Thr Gly Ala Asp Phe Ser Leu Asn
   225                 230                 235                 240
   Gly Gly Leu His Met Gly
                   245
<210> 7
   <211> 36
   <212> DNA
   <213> Artificial Sequence
<220>
   <223> Description of Artificial Sequence: oligonucleotide primer-B1L-Bam
<400> 7
   cgcggatcca tgactcagcg cattgcgtat gtgacc 36
<210> 8
   <211> 37
   <212> DNA
   <213> Artificial Sequence
<220>
   <223> Description of Artificial Sequence: oligonucleotide primer- B1L-Xba
<400> 8
   gctctagatc agcccatatg caggccgccg ttgagcg 37
<210> 9
   <211> 1926
   <212> DNA
   <213> Alcaligenes eutrophus
<220>
   <221> misc_feature
   <222> (1)..(1926)
   <223> phbA-linker-phbB fusion gene
<400> 9
   atgactgacg ttgtcatcgt atccgccgcc cgcaccgcgg tcggcaagtt tggcggctcg 60
   ctggccaaga tcccggcacc ggaactgggt gccgtggtca tcaaggccgc gctggagcgc 120
   gccggcgtca agccggagca ggtgagcgaa gtcatcatgg gccaggtgct gaccgccggt 180
   tcgggccaga accccgcacg ccaggccgcg atcaaggccg gcctgccggc gatggtgccg 240
   gccatgacca tcaacaaggt gtgcggctcg ggcctgaagg ccgtgatgct ggccgccaac 300
   gcgatcatgg cgggcgacgc cgagatcgtg gtggccggcg gccaggaaaa catgagcgcc 360
   gccccgcacg tgctgccggg ctcgcgcgat ggtttccgca tgggcgatgc caagctggtc 420
   gacaccatga tcgtcgacgg cctgtgggac gtgtacaacc agtaccacat gggcatcacc 480
   gccgagaacg tggccaagga atacggcatc acacgcgagg cgcaggatga gttcgccgtc 540
   ggctcgcaga acaaggccga agccgcgcag aaggccggca agtttgacga agagatcgtc 600
   ccggtgctga tcccgcagcg caagggcgac ccggtggcct tcaagaccga cgagttcgtg 660
   cgccagggcg ccacgctgga cagcatgtcc ggcctcaagc ccgccttcga caaggccggc 720
   acggtgaccg cggccaacgc ctcgggcctg aacgacggcg ccgccgcggt ggtggtgatg 780
   tcggcggcca aggccaagga actgggcctg accccgctgg ccacgatcaa gagctatgcc 840
   aacgccggtg tcgatcccaa ggtgatgggc atgggcccgg tgccggcctc caagcgcgcc 900
   ctgtcgcgcg ccgagtggac cccgcaagac ctggacctga tggagatcaa cgaggccttt 960
   gccgcgcagg cgctggcggt gcaccagcag atgggctggg acacctccaa ggtcaatgtg 1020
   aacggcggcg ccatcgccat cggccacccg atcggcgcgt cgggctgccg tatcctggtg 1080
   acgctgctgc acgagatgaa gcgccgtgac gcgaagaagg gcctggcctc gctgtgcatc 1140
   ggcggcggca tgggcgtggc gctggcagtc gagcgcaaag gatccatgac tcagcgcatt 1200
   gcgtatgtga ccggcggcat gggtggtatc ggaaccgcca tttgccagcg gctggccaag 1260
   gatggctttc gtgtggtggc cggttgcggc cccaactcgc cgcgccgcga aaagtggctg 1320
   gagcagcaga aggccctggg cttcgatttc attgcctcgg aaggcaatgt ggctgactgg 1380
   gactcgacca agaccgcatt cgacaaggtc aagtccgagg tcggcgaggt tgatgtgctg 1440
   atcaacaacg ccggtatcac ccgcgacgtg gtgttccgca agatgacccg cgccgactgg 1500
   gatgcggtga tcgacaccaa cctgacctcg ctgttcaacg tcaccaagca ggtgatcgac 1560
   ggcatggccg accgtggctg gggccgcatc gtcaacatct cgtcggtgaa cgggcagaag 1620
   ggccagttcg gccagaccaa ctactccacc gccaaggccg gcctgcatgg cttcaccatg 1680
   gcactggcgc aggaagtggc gaccaagggc gtgaccgtca acacggtctc tccgggctat 1740
   atcgccaccg acatggtcaa ggcgatccgc caggacgtgc tcgacaagat cgtcgcgacg 1800
   atcccggtca agcgcctggg cctgccggaa gagatcgcct cgatctgcgc ctggttgtcg 1860
   tcggaggagt ccggtttctc gaccggcgcc gacttctcgc tcaacggcgg cctgcatatg 1920
   ggctga 1926
<210> 10
   <211> 641
   <212> PRT
   <213> Artificial Sequence
<220>
   <223> Description of Artificial Sequence: Thredase Fusion Protein
<220>
   <221> PEPTIDE
   <222> (1)..(641)
<400> 10
   Met Thr Asp Val Val Ile Val Ser Ala Ala Arg Thr Ala Val Gly Lys
   1               5                   10                  15
   Phe Gly Gly Ser Leu Ala Lys Ile Pro Ala Pro Glu Leu Gly Ala Val
               20                  25                  30
   Val Ile Lys Ala Ala Leu Glu Arg Ala Gly Val Lys Pro Glu Gln Val
           35                  40                  45
   Ser Glu Val Ile Met Gly Gln Val Leu Thr Ala Gly Ser Gly Gln Asn
       50                  55                  60
   Pro Ala Arg Gln Ala Ala Ile Lys Ala Gly Leu Pro Ala Met Val Pro
   65                  70                  75                  80
   Ala Met Thr Ile Asn Lys Val Cys Gly Ser Gly Leu Lys Ala Val Met
                   85                  90                  95
   Leu Ala Ala Asn Ala Ile Met Ala Gly Asp Ala Glu Ile Val Val Ala
               100                 105                 110
   Gly Gly Gln Glu Asn Met Ser Ala Ala Pro His Val Leu Pro Gly Ser
           115                 120                 125
   Arg Asp Gly Phe Arg Met Gly Asp Ala Lys Leu Val Asp Thr Met Ile
       130                 135                 140
   Val Asp Gly Leu Trp Asp Val Tyr Asn Gln Tyr His Met Gly Ile Thr
   145                 150                 155                 160
   Ala Glu Asn Val Ala Lys Glu Tyr Gly Ile Thr Arg Glu Ala Gln Asp
                   165                 170                 175
   Glu Phe Ala Val Gly Ser Gln Asn Lys Ala Glu Ala Ala Gln Lys Ala
               180                 185                 190
   Gly Lys Phe Asp Glu Glu Ile Val Pro Val Leu Ile Pro Gln Arg Lys
           195                 200                 205
   Gly Asp Pro Val Ala Phe Lys Thr Asp Glu Phe Val Arg Gln Gly Ala
       210                 215                 220
   Thr Leu Asp Ser Met Ser Gly Leu Lys Pro Ala Phe Asp Lys Ala Gly
   225                 230                 235                 240
   Thr Val Thr Ala Ala Asn Ala Ser Gly Leu Asn Asp Gly Ala Ala Ala
                   245                 250                 255
   Val Val Val Met Ser Ala Ala Lys Ala Lys Glu Leu Gly Leu Thr Pro
   260 265 270
   Leu Ala Thr Ile Lys Ser Tyr Ala Asn Ala Gly Val Asp Pro Lys Val
   275 280 285
   Met Gly Met Gly Pro Val Pro Ala Ser Lys Arg Ala Leu Ser Arg Ala
   290 295 300
   Glu Trp Thr Pro Gln Asp Leu Asp Leu Met Glu Ile Asn Glu Ala Phe
   305 310 315 320
   Ala Ala Gln Ala Leu Ala Val His Gln Gln Met Gly Trp Asp Thr Ser
   325 330 335
   Lys Val Asn Val Asn Gly Gly Ala Ile Ala Ile Gly His Pro Ile Gly
   340 345 350
   Ala Ser Gly Cys Arg Ile Leu Val Thr Leu Leu His Glu Met Lys Arg
   355 360 365
   Arg Asp Ala Lys Lys Gly Leu Ala Ser Leu Cys Ile Gly Gly Gly Met
   370 375 380
   Gly Val Ala Leu Ala Val Glu Arg Lys Gly Ser Met Thr Gln Arg Ile
   385 390 395 400
   Ala Tyr Val Thr Gly Gly Met Gly Gly Ile Gly Thr Ala Ile Cys Gln
   405 410 415
   Arg Leu Ala Lys Asp Gly Phe Arg Val Val Ala Gly Cys Gly Pro Asn
   420 425 430
   Ser Pro Arg Arg Glu Lys Trp Leu Glu Gln Gln Lys Ala Leu Gly Phe
   435 440 445
   Asp Phe Ile Ala Ser Glu Gly Asn Val Ala Asp Trp Asp Ser Thr Lys
   450 455 460
   Thr Ala Phe Asp Lys Val Lys Ser Glu Val Gly Glu Val Asp Val Leu
   465 470 475 480
   Ile Asn Asn Ala Gly Ile Thr Arg Asp Val Val Phe Arg Lys Met Thr
   485 490 495
   Arg Ala Asp Trp Asp Ala Val Ile Asp Thr Asn Leu Thr Ser Leu Phe
   500 505 510
   Asn Val Thr Lys Gln Val Ile Asp Gly Met Ala Asp Arg Gly Trp Gly
   515 520 525
   Arg Ile Val Asn Ile Ser Ser Val Asn Gly Gln Lys Gly Gln Phe Gly
   530 535 540
   Gln Thr Asn Tyr Ser Thr Ala Lys Ala Gly Leu His Gly Phe Thr Met
   545 550 555 560
   Ala Leu Ala Gln Glu Val Ala Thr Lys Gly Val Thr Val Asn Thr Val
   565 570 575
   Ser Pro Gly Tyr Ile Ala Thr Asp Met Val Lys Ala Ile Arg Gln Asp
   580 585 590
   Val Leu Asp Lys Ile Val Ala Thr Ile Pro Val Lys Arg Leu Gly Leu
   595 600 605
   Pro Glu Glu Ile Ala Ser Ile Cys Ala Trp Leu Ser Ser Glu Glu Ser
   610 615 620
   Gly Phe Ser Thr Gly Ala Asp Phe Ser Leu Asn Gly Gly Leu His Met
   625 630 635 640
   Gly
<210> 11
   <211> 9
   <212> DNA
   <213> Artificial Sequence
<220>
   <223> Description of Artificial Sequence: oligonucleotide primer- L5A
<400> 11
   gatctaccg 9
<210> 12
   <211> 9
   <212> DNA
   <213> Artificial Sequence
<220>
   <223> Description of Artificial Sequence: oligonucleotide primer- L5B
<400> 12
   atggcctag 9
<210> 13
   <211> 5
   <212> PRT
   <213> Artificial Sequence
<220>
   <223> Description of Artificial Sequence: Peptide Linker
<220>
   <221> PEPTIDE
   <222> (1)..(5)
<400> 13
   Gly Ser Thr Gly Ser
   1 5
<210> 14
   <211> 46
   <212> DNA
   <213> Artificial Sequence
<220>
   <223> Description of Artificial Sequence: oligonucleotide primer- BlF-Kpn
<400> 14
   ggggtaccag gaggttttta tgactcagcg cattgcgtat gtgacc 46
<210> 15
   <211> 35
   <212> DNA
   <213> Artificial Sequence
<220>
   <223> Description of Artificial Sequence: oligonucleotide primer- B1F-BamHI
<400> 15
   cgcggatccg cccatatgca ggccgccgtt gagcg 35
<210> 16
   <211> 33
   <212> DNA
   <213> Alcaligenes eutrophus
<220>
   <223> Description of Artificial Sequence: oligonucleotide primer- A1L BamHI
<400> 16
   cgcggatcca tgactgacgt tgtcatcgta tcc 33
<210> 17
   <211> 39
   <212> DNA
   <213> Artificial Sequence
<220>
   <223> Description of Artificial Sequence: oligonucleotide primer- A1L-XbaI
<400> 17
   gctctagatt atttgcgctc gactgccagc gccacgccc 39
<210> 18
   <211> 1926
   <212> DNA
   <213> Alcaligenes eutrophus
<220>
   <221> gene
   <222> (1)..(1926)
   <223> phbB-linker-phbA fusion gene
<400> 18
   atgactcagc gcattgcgta tgtgaccggc ggcatgggtg gtatcggaac cgccatttgc 60
   cagcggctgg ccaaggatgg ctttcgtgtg gtggccggtt gcggccccaa ctcgccgcgc 120
   cgcgaaaagt ggctggagca gcagaaggcc ctgggcttcg atttcattgc ctcggaaggc 180
   aatgtggctg actgggactc gaccaagacc gcattcgaca aggtcaagtc cgaggtcggc 240
   gaggttgatg tgctgatcaa caacgccggt atcacccgcg acgtggtgtt ccgcaagatg 300
   acccgcgccg actgggatgc ggtgatcgac accaacctga cctcgctgtt caacgtcacc 360
   aagcaggtga tcgacggcat ggccgaccgt ggctggggcc gcatcgtcaa catctcgtcg 420
   gtgaacgggc agaagggcca gttcggccag accaactact ccaccgccaa ggccggcctg 480
   catggcttca ccatggcact ggcgcaggaa gtggcgacca agggcgtgac cgtcaacacg 540
   gtctctccgg gctatatcgc caccgacatg gtcaaggcga tccgccagga cgtgctcgac 600
   aagatcgtcg cgacgatccc ggtcaagcgc ctgggcctgc cggaagagat cgcctcgatc 660
   tgcgcctggt tgtcgtcgga ggagtccggt ttctcgaccg gcgccgactt ctcgctcaac 720
   ggcggcctgc atatgggcgg atccatgact gacgttgtca tcgtatccgc cgcccgcacc 780
   gcggtcggca agtttggcgg ctcgctggcc aagatcccgg caccggaact gggtgccgtg 840
   gtcatcaagg ccgcgctgga gcgcgccggc gtcaagccgg agcaggtgag cgaagtcatc 900
   atgggccagg tgctgaccgc cggttcgggc cagaaccccg cacgccaggc cgcgatcaag 960
   gccggcctgc cggcgatggt gccggccatg accatcaaca aggtgtgcgg ctcgggcctg 1020
   aaggccgtga tgctggccgc caacgcgatc atggcgggcg acgccgagat cgtggtggcc 1080
   ggcggccagg aaaacatgag cgccgccccg cacgtgctgc cgggctcgcg cgatggtttc 1140
   cgcatgggcg atgccaagct ggtcgacacc atgatcgtcg acggcctgtg ggacgtgtac 1200
   aaccagtacc acatgggcat caccgccgag aacgtggcca aggaatacgg catcacacgc 1260
   gaggcgcagg atgagttcgc cgtcggctcg cagaacaagg ccgaagccgc gcagaaggcc 1320
   ggcaagtttg acgaagagat cgtcccggtg ctgatcccgc agcgcaaggg cgacccggtg 1380
   gccttcaaga ccgacgagtt cgtgcgccag ggcgccacgc tggacagcat gtccggcctc 1440
   aagcccgcct tcgacaaggc cggcacggtg accgcggcca acgcctcggg cctgaacgac 1500
   ggcgccgccg cggtggtggt gatgtcggcg gccaaggcca aggaactggg cctgaccccg 1560
   ctggccacga tcaagagcta tgccaacgcc ggtgtcgatc ccaaggtgat gggcatgggc 1620
   ccggtgccgg cctccaagcg cgccctgtcg cgcgccgagt ggaccccgca agacctggac 1680
   ctgatggaga tcaacgaggc ctttgccgcg caggcgctgg cggtgcacca gcagatgggc 1740
   tgggacacct ccaaggtcaa tgtgaacggc ggcgccatcg ccatcggcca cccgatcggc 1800
   gcgtcgggct gccgtatcct ggtgacgctg ctgcacgaga tgaagcgccg tgacgcgaag 1860
   aagggcctgg cctcgctgtg catcggcggc ggcatgggcg tggcgctggc agtcgagcgc 1920
   aaataa                                                            1926
<210> 19
   <211> 641
   <212> PRT
   <213> Artificial Sequence
<220>
   <223> Description of Artificial Sequence: Reducthase Fusion Protein
<220>
   <221> PEPTIDE
   <222> (1)..(641)
<400> 19
   Met Thr Gln Arg Ile Ala Tyr Val Thr Gly Gly Met Gly Gly Ile Gly
   1               5                   10                  15
   Thr Ala Ile Cys Gln Arg Leu Ala Lys Asp Gly Phe Arg Val Val Ala
               20                  25                  30
   Gly Cys Gly Pro Asn Ser Pro Arg Arg Glu Lys Trp Leu Glu Gln Gln
           35                  40                  45
   Lys Ala Leu Gly Phe Asp Phe Ile Ala Ser Glu Gly Asn Val Ala Asp
           50                  55                  60
   Trp Asp Ser Thr Lys Thr Ala Phe Asp Lys Val Lys Ser Glu Val Gly
    65                  70                  75                  80
   Glu Val Asp Val Leu Ile Asn Asn Ala Gly Ile Thr Arg Asp Val Val
                    85                  90                  95
   Phe Arg Lys Met Thr Arg Ala Asp Trp Asp Ala Val Ile Asp Thr Asn
               100                 105                 110
   Leu Thr Ser Leu Phe Asn Val Thr Lys Gln Val Ile Asp Gly Met Ala
           115                 120                 125
   Asp Arg Gly Trp Gly Arg Ile Val Asn Ile Ser Ser Val Asn Gly Gln
       130                 135                 140
   Lys Gly Gln Phe Gly Gln Thr Asn Tyr Ser Thr Ala Lys Ala Gly Leu
   145                 150                 155                 160
   His Gly Phe Thr Met Ala Leu Ala Gln Glu Val Ala Thr Lys Gly Val
                   165                 170                 175
   Thr Val Asn Thr Val Ser Pro Gly Tyr Ile Ala Thr Asp Met Val Lys
               180                 185                 190
   Ala Ile Arg Gln Asp Val Leu Asp Lys Ile Val Ala Thr Ile Pro Val
           195                 200                 205
   Lys Arg Leu Gly Leu Pro Glu Glu Ile Ala Ser Ile Cys Ala Trp Leu
       210                 215                 220
   Ser Ser Glu Glu Ser Gly Phe Ser Thr Gly Ala Asp Phe Ser Leu Asn
   225                 230                 235                 240
   Gly Gly Leu His Met Gly Gly Ser Met Thr Asp Val Val Ile Val Ser
                   245                 250                 255
   Ala Ala Arg Thr Ala Val Gly Lys Phe Gly Gly Ser Leu Ala Lys Ile
               260                 265                 270
   Pro Ala Pro Glu Leu Gly Ala Val Val Ile Lys Ala Ala Leu Glu Arg
           275                 280                 285
   Ala Gly Val Lys Pro Glu Gln Val Ser Glu Val Ile Met Gly Gln Val
       290                 295                 300
   Leu Thr Ala Gly Ser Gly Gln Asn Pro Ala Arg Gln Ala Ala Ile Lys
   305 310 315 320
   Ala Gly Leu Pro Ala Met Val Pro Ala Met Thr Ile Asn Lys Val Cys
   325 330 335
   Gly Ser Gly Leu Lys Ala Val Met Leu Ala Ala Asn Ala Ile Met Ala
   340 345 350
   Gly Asp Ala Glu Ile Val Val Ala Gly Gly Gln Glu Asn Met Ser Ala
   355 360 365
   Ala Pro His Val Leu Pro Gly Ser Arg Asp Gly Phe Arg Met Gly Asp
   370 375 380
   Ala Lys Leu Val Asp Thr Met Ile Val Asp Gly Leu Trp Asp Val Tyr
   385 390 395 400
   Asn Gln Tyr His Met Gly Ile Thr Ala Glu Asn Val Ala Lys Glu Tyr
   405 410 415
   Gly Ile Thr Arg Glu Ala Gln Asp Glu Phe Ala Val Gly Ser Gln Asn
   420 425 430
   Lys Ala Glu Ala Ala Gln Lys Ala Gly Lys Phe Asp Glu Glu Ile Val
   435 440 445
   Pro Val Leu Ile Pro Gln Arg Lys Gly Asp Pro Val Ala Phe Lys Thr
   450 455 460
   Asp Glu Phe Val Arg Gln Gly Ala Thr Leu Asp Ser Met Ser Gly Leu
   465 470 475 480
   Lys Pro Ala Phe Asp Lys Ala Gly Thr Val Thr Ala Ala Asn Ala Ser
   485 490 495
   Gly Leu Asn Asp Gly Ala Ala Ala Val Val Val Met Ser Ala Ala Lys
   500 505 510
   Ala Lys Glu Leu Gly Leu Thr Pro Leu Ala Thr Ile Lys Ser Tyr Ala
   515 520 525
   Asn Ala Gly Val Asp Pro Lys Val Met Gly Met Gly Pro Val Pro Ala
   530 535 540
   Ser Lys Arg Ala Leu Ser Arg Ala Glu Trp Thr Pro Gln Asp Leu Asp
   545 550 555 560
   Leu Met Glu Ile Asn Glu Ala Phe Ala Ala Gln Ala Leu Ala Val His
                   565                 570                 575
   Gln Gln Met Gly Trp Asp Thr Ser Lys Val Asn Val Asn Gly Gly Ala
               580                 585                 590
   Ile Ala Ile Gly His Pro Ile Gly Ala Ser Gly Cys Arg Ile Leu Val
           595                 600                 605
   Thr Leu Leu His Glu Met Lys Arg Arg Asp Ala Lys Lys Gly Leu Ala
       610                 615                 620
   Ser Leu Cys Ile Gly Gly Gly Met Gly Val Ala Leu Ala Val Glu Arg
   625                 630                 635                 640
   Lys
<210> 20
   <211> 1680
   <212> DNA
   <213> Pseudomonas oleovorans
<220>
   <221> gene
   <222> (1)..(1680)
   <223> phbCl gene
<400> 20
   atgagtaaca agaacaacga tgagctgcag cggcaggcct cggaaaacac cctggggctg 60
   aacccggtca tcggtatccg ccgcaaagac ctgttgagct cggcacgcac cgtgctgcgc 120
   caggccgtgc gccaaccgct gcacagcgcc aagcatgtgg cccactttgg cctggagctg 180
   aagaacgtgc tgctgggcaa gtccagcctt gccccggaaa gcgacgaccg tcgcttcaat 240
   gacccggcat ggagcaacaa cccactttac cgccgctacc tgcaaaccta tctggcctgg 300
   cgcaaggagc tgcaggactg gatcggcaac agcgacctgt cgccccagga catcagccgc 360
   ggccagttcg tcatcaacct gatgaccgaa gccatggctc cgaccaacac cctgtccaac 420
   ccggcagcag tcaaacgctt cttcgaaacc ggcggcaaga gcctgctcga tggcctgtcc 480
   aacctggcca aggacctggt caacaacggt ggcatgccca gccaggtgaa catggacgcc 540
   ttcgaggtgg gcaagaacct gggcaccagt gaaggcgccg tggtgtaccg caacgatgtg 600
   ctggagctga tccagtacaa gcccatcacc gagcaggtgc atgcccgccc gctgctggtg 660
   gtgccgccgc agatcaacaa gttctacgta ttcgacctga gcccggaaaa gagcctggca 720
   cgctactgcc tgcgctcgca gcagcagacc ttcatcatca gctggcgcaa cccgaccaaa 780
   gcccagcgcg aatggggcct gtccacctac atcgacgcgc tcaaggaggc ggtcgacgcg 840
   gtgctggcga ttaccggcag caaggacctg aacatgctcg gtgcctgctc cggcggcatc 900
   acctgcacgg cattggtcgg ccactatgcc gccctcggcg aaaacaaggt caatgccctg 960
   accctgctgg tcagcgtgct ggacaccacc atggacaacc aggtcgccct gttcgtcgac 1020
   gagcagactt tggaggccgc caagcgccac tcctaccagg ccggtgtgct cgaaggcagc 1080
   gagatggcca aggtgttcgc ctggatgcgc cccaacgacc tgatctggaa ctactgggtc 1140
   aacaactacc tgctcggcaa cgagccgccg gtgttcgaca tcctgttctg gaacaacgac 1200
   accacgcgcc tgccggccgc cttccacggc gacctgatcg aaatgttcaa gagcaacccg 1260
   ctgacccgcc cggacgccct ggaggtttgc ggcactccga tcgacctgaa acaggtcaaa 1320
   tgcgacatct acagccttgc cggcaccaac gaccacatca ccccgtggca gtcatgctac 1380
   cgctcggcgc acctgttcgg cggcaagatc gagttcgtgc tgtccaacag cggccacatc 1440
   cagagcatcc tcaacccgcc aggcaacccc aaggcgcgct tcatgaccgg tgccgatcgc 1500
   ccgggtgacc cggtggcctg gcaggaaaac gccaccaagc atgccgactc ctggtggctg 1560
   cactggcaaa gctggctggg cgagcgtgcc ggcgagctgg aaaaggcgcc gacccgcctg 1620
   ggcaaccgtg cctatgccgc tggcgaggca tccccgggca cctacgttca cgagcgttga 1680
<210> 21
   <211> 559
   <212> PRT
   <213> Pseudomonas oleovorans
<220>
   <221> PEPTIDE
   <222> (1)..(559)
   <223> PHA Polymerase
<400> 21
   Met Ser Asn Lys Asn Asn Asp Glu Leu Gln Arg Gln Ala Ser Glu Asn
   1               5                   10                  15
   Thr Leu Gly Leu Asn Pro Val Ile Gly Ile Arg Arg Lys Asp Leu Leu
               20                  25                  30
   Ser Ser Ala Arg Thr Val Leu Arg Gln Ala Val Arg Gln Pro Leu His
           35                  40                  45
   Ser Ala Lys His Val Ala His Phe Gly Leu Glu Leu Lys Asn Val Leu
       50                  55                  60
   Leu Gly Lys Ser Ser Leu Ala Pro Glu Ser Asp Asp Arg Arg Phe Asn
   65                  70                  75                  80
   Asp Pro Ala Trp Ser Asn Asn Pro Leu Tyr Arg Arg Tyr Leu Gln Thr
                   85                  90                  95
   Tyr Leu Ala Trp Arg Lys Glu Leu Gln Asp Trp Ile Gly Asn Ser Asp
               100                 105                 110
   Leu Ser Pro Gln Asp Ile Ser Arg Gly Gln Phe Val Ile Asn Leu Met
           115                 120                 125
   Thr Glu Ala Met Ala Pro Thr Asn Thr Leu Ser Asn Pro Ala Ala Val
   130 135 140
   Lys Arg Phe Phe Glu Thr Gly Gly Lys Ser Leu Leu Asp Gly Leu Ser
   145 150 155 160
   Asn Leu Ala Lys Asp Leu Val Asn Asn Gly Gly Met Pro Ser Gln Val
   165 170 175
   Asn Met Asp Ala Phe Glu Val Gly Lys Asn Leu Gly Thr Ser Glu Gly
   180 185 190
   Ala Val Val Tyr Arg Asn Asp Val Leu Glu Leu Ile Gln Tyr Lys Pro
   195 200 205
   Ile Thr Glu Gln Val His Ala Arg Pro Leu Leu Val Val Pro Pro Gln
   210 215 220
   Ile Asn Lys Phe Tyr Val Phe Asp Leu Ser Pro Glu Lys Ser Leu Ala
   225 230 235 240
   Arg Tyr Cys Leu Arg Ser Gln Gln Gln Thr Phe Ile Ile Ser Trp Arg
   245 250 255
   Asn Pro Thr Lys Ala Gln Arg Glu Trp Gly Leu Ser Thr Tyr Ile Asp
   260 265 270
   Ala Leu Lys Glu Ala Val Asp Ala Val Leu Ala Ile Thr Gly Ser Lys
   275 280 285
   Asp Leu Asn Met Leu Gly Ala Cys Ser Gly Gly Ile Thr Cys Thr Ala
   290 295 300
   Leu Val Gly His Tyr Ala Ala Leu Gly Glu Asn Lys Val Asn Ala Leu
   305 310 315 320
   Thr Leu Leu Val Ser Val Leu Asp Thr Thr Met Asp Asn Gln Val Ala
   325 330 335
   Leu Phe Val Asp Glu Gln Thr Leu Glu Ala Ala Lys Arg His Ser Tyr
   340 345 350
   Gln Ala Gly Val Leu Glu Gly Ser Glu Met Ala Lys Val Phe Ala Trp
   355 360 365
   Met Arg Pro Asn Asp Leu Ile Trp Asn Tyr Trp Val Asn Asn Tyr Leu
   370 375 380
   Leu Gly Asn Glu Pro Pro Val Phe Asp Ile Leu Phe Trp Asn Asn Asp
   385 390 395 400
   Thr Thr Arg Leu Pro Ala Ala Phe His Gly Asp Leu Ile Glu Met Phe
   405 410 415
   Lys Ser Asn Pro Leu Thr Arg Pro Asp Ala Leu Glu Val Cys Gly Thr
   420 425 430
   Pro Ile Asp Leu Lys Gln Val Lys Cys Asp Ile Tyr Ser Leu Ala Gly
   435 440 445
   Thr Asn Asp His Ile Thr Pro Trp Gln Ser Cys Tyr Arg Ser Ala His
   450 455 460
   Leu Phe Gly Gly Lys Ile Glu Phe Val Leu Ser Asn Ser Gly His Ile
   465 470 475 480
   Gln Ser Ile Leu Asn Pro Pro Gly Asn Pro Lys Ala Arg Phe Met Thr
   485 490 495
   Gly Ala Asp Arg Pro Gly Asp Pro Val Ala Trp Gln Glu Asn Ala Thr
   500 505 510
   Lys His Ala Asp Ser Trp Trp Leu His Trp Gln Ser Trp Leu Gly Glu
   515 520 525
   Arg Ala Gly Glu Leu Glu Lys Ala Pro Thr Arg Leu Gly Asn Arg Ala
   530 535 540
   Tyr Ala Ala Gly Glu Ala Ser Pro Gly Thr Tyr Val His Glu Arg
   545 550 555
<210> 22
   <211> 42
   <212> DNA
   <213> Artificial Sequence
<220>
   <223> Description of Artificial Sequence: oligonucleotide primer- C3 up I
<400> 22
   ggaattcagg aggttttatg agtaacaaga acaacgatga gc 42
<210> 23
   <211> 30
   <212> DNA
   <213> Artificial Sequence
<220>
   <223> Description of Artificial Sequence: oligonucleotide primer- C3 up II
<400> 23
   cgggatccac gctcgtgaac gtaggtgccc 30
<210> 24
   <211> 30
   <212> DNA
   <213> Artificial Sequence
<220>
   <223> Description of Artificial Sequence: oligonucleotide primer- C3 dw I
<400> 24
   cgggatccag taacaagaac aacgatgagc 30
<210> 25
   <211> 38
   <212> DNA
   <213> Artificial Sequence
<220>
   <223> Description of Artificial Sequence: oligonucleotide primer- C3 dw II
<400> 25
   gctctagaag ctttcaacgc tcgtgaacgt aggtgccc 38
<210> 26
   <211> 888
   <212> DNA
   <213> Pseudomonas putida
<220>
   <221> gene
   <222> (1)..(888)
   <223> phaG
<400> 26
   atgaggccag aaatcgctgt acttgatatc caaggtcagt atcgggttta cacggagttc 60
   tatcgcgcgg atgcggccga aaacacgatc atcctgatca acggctcgct ggccaccacg 120
   gcctcgttcg cccagacggt acgtaacctg cacccacagt tcaacgtggt tctgttcgac 180
   cagccgtatt caggcaagtc caagccgcac aaccgtcagg aacggctgat cagcaaggag 240
   accgaggcgc atatcctcct tgagctgatc gagcacttcc aggcagacca cgtgatgtct 300
   ttttcgtggg gtggcgcaag cacgctgctg gcgctggcgc accagccgcg gtacgtgaag 360
   aaggcagtgg tgagttcgtt ctcgccagtg atcaacgagc cgatgcgcga ctatctggac 420
   cgtggctgcc agtacctggc cgcctgcgac cgttatcagg tcggcaacct ggtcaatgac 480
   accatcggca agcacttgcc gtcgctgttc aaacgcttca actaccgcca tgtgagcagc 540
   ctggacagcc acgagtacgc acagatgcac ttccacatca accaggtgct ggagcacgac 600
   ctggaacgtg cgctgcaagg cgcgcgcaat atcaacatcc cggtgctgtt catcaacggc 660
   gagcgcgacg agtacaccac agtcgaggat gcgcggcagt tcagcaagca tgtgggcaga 720
   agccagttca gcgtgatccg cgatgcgggc cacttcctgg acatggagaa caagaccgcc 780
   tgcgagaaca cccgcaatgt catgctgggc ttcctcaagc caaccgtgcg tgaaccccgc 840
   caacgttacc aacccgtgca gcaggggcag catgcatttg ccatctga             888
<210> 27
   <211> 295
   <212> PRT
   <213> Artificial Sequence
<220>
   <223> Description of Artificial Sequence: acyl ACP-CoA transferase
<220>
   <221> PEPTIDE
   <222> (1)..(295)
<400> 27
   Met Arg Pro Glu Ile Ala Val Leu Asp Ile Gln Gly Gln Tyr Arg Val
   1               5                   10                  15
   Tyr Thr Glu Phe Tyr Arg Ala Asp Ala Ala Glu Asn Thr Ile Ile Leu
               20                  25                  30
   Ile Asn Gly Ser Leu Ala Thr Thr Ala Ser Phe Ala Gln Thr Val Arg
           35                  40                  45
   Asn Leu His Pro Gln Phe Asn Val Val Leu Phe Asp Gln Pro Tyr Ser
       50                  55                  60
   Gly Lys Ser Lys Pro His Asn Arg Gln Glu Arg Leu Ile Ser Lys Glu
   65                  70                  75                  80
   Thr Glu Ala His Ile Leu Leu Glu Leu Ile Glu His Phe Gln Ala Asp
                   85                  90                  95
   His Val Met Ser Phe Ser Trp Gly Gly Ala Ser Thr Leu Leu Ala Leu
   100 105 110
   Ala His Gln Pro Arg Tyr Val Lys Lys Ala Val Val Ser Ser Phe Ser
   115 120 125
   Pro Val Ile Asn Glu Pro Met Arg Asp Tyr Leu Asp Arg Gly Cys Gln
   130 135 140
   Tyr Leu Ala Ala Cys Asp Arg Tyr Gln Val Gly Asn Leu Val Asn Asp
   145 150 155 160
   Thr Ile Gly Lys His Leu Pro Ser Leu Phe Lys Arg Phe Asn Tyr Arg
   165 170 175
   His Val Ser Ser Leu Asp Ser His Glu Tyr Ala Gln Met His Phe His
   180 185 190
   Ile Asn Gln Val Leu Glu His Asp Leu Glu Arg Ala Leu Gln Gly Ala
   195 200 205
   Arg Asn Ile Asn Ile Pro Val Leu Phe Ile Asn Gly Glu Arg Asp Glu
   210 215 220
   Tyr Thr Thr Val Glu Asp Ala Arg Gln Phe Ser Lys His Val Gly Arg
   225 230 235 240
   Ser Gln Phe Ser Val Ile Arg Asp Ala Gly His Phe Leu Asp Met Glu
   245 250 255
   Asn Lys Thr Ala Cys Glu Asn Thr Arg Asn Val Met Leu Gly Phe Leu
   260 265 270
   Lys Pro Thr Val Arg Glu Pro Arg Gln Arg Tyr Gln Pro Val Gln Gln
   275 280 285
   Gly Gln His Ala Phe Ala Ile
   290 295
<210> 28
   <211> 30
   <212> DNA
   <213> Artificial Sequence
<220>
   <223> Description of Artificial Sequence: oligonucleotide primer- G3 dw I
<400> 28
   cgggatccag gccagaaatc gctgtacttg 30
<210> 29
   <211> 38
   <212> DNA
   <213> Artificial Sequence
<220>
   <223> Description of Artificial Sequence: oligonucleotide primer- G3 dw II
<400> 29
   gctctagaag ctttcagatg gcaaatgcat gctgcccc 38
<210> 30
   <211> 42
   <212> DNA
   <213> Artificial Sequence
<220>
   <223> Description of Artificial Sequence: oligonucleotide primer- G3 up I
<400> 30
   ggaattcagg aggttttatg aggccagaaa tcgctgtact tg 42
<210> 31
   <211> 30
   <212> DNA
   <213> Artificial Sequence
<220>
   <223> Description of Artificial Sequence: oligonucleotide primer- G3 up II
<400> 31
   cgggatccga tggcaaatgc atgctgcccc 30
<210> 32
   <211> 2571
   <212> DNA
   <213> Pseudomonas putida
<220>
   <221> gene
   <222> (1)..(2571)
   <223> phaCl-linker-phaG fusion gene
<400> 32
   atgagtaaca agaacaacga tgagctgcag cggcaggcct cggaaaacac cctggggctg 60
   aacccggtca tcggtatccg ccgcaaagac ctgttgagct cggcacgcac cgtgctgcgc 120
   caggccgtgc gccaaccgct gcacagcgcc aagcatgtgg cccactttgg cctggagctg 180
   aagaacgtgc tgctgggcaa gtccagcctt gccccggaaa gcgacgaccg tcgcttcaat 240
   gacccggcat ggagcaacaa cccactttac cgccgctacc tgcaaaccta tctggcctgg 300
   cgcaaggagc tgcaggactg gatcggcaac agcgacctgt cgccccagga catcagccgc 360
   ggccagttcg tcatcaacct gatgaccgaa gccatggctc cgaccaacac cctgtccaac 420
   ccggcagcag tcaaacgctt cttcgaaacc ggcggcaaga gcctgctcga tggcctgtcc 480
   aacctggcca aggacctggt caacaacggt ggcatgccca gccaggtgaa catggacgcc 540
   ttcgaggtgg gcaagaacct gggcaccagt gaaggcgccg tggtgtaccg caacgatgtg 600
   ctggagctga tccagtacaa gcccatcacc gagcaggtgc atgcccgccc gctgctggtg 660
   gtgccgccgc agatcaacaa gttctacgta ttcgacctga gcccggaaaa gagcctggca 720
   cgctactgcc tgcgctcgca gcagcagacc ttcatcatca gctggcgcaa cccgaccaaa 780
   gcccagcgcg aatggggcct gtccacctac atcgacgcgc tcaaggaggc ggtcgacgcg 840
   gtgctggcga ttaccggcag caaggacctg aacatgctcg gtgcctgctc cggcggcatc 900
   acctgcacgg cattggtcgg ccactatgcc gccctcggcg aaaacaaggt caatgccctg 960
   accctgctgg tcagcgtgct ggacaccacc atggacaacc aggtcgccct gttcgtcgac 1020
   gagcagactt tggaggccgc caagcgccac tcctaccagg ccggtgtgct cgaaggcagc 1080
   gagatggcca aggtgttcgc ctggatgcgc cccaacgacc tgatctggaa ctactgggtc 1140
   aacaactacc tgctcggcaa cgagccgccg gtgttcgaca tcctgttctg gaacaacgac 1200
   accacgcgcc tgccggccgc cttccacggc gacctgatcg aaatgttcaa gagcaacccg 1260
   ctgacccgcc cggacgccct ggaggtttgc ggcactccga tcgacctgaa acaggtcaaa 1320
   tgcgacatct acagccttgc cggcaccaac gaccacatca ccccgtggca gtcatgctac 1380
   cgctcggcgc acctgttcgg cggcaagatc gagttcgtgc tgtccaacag cggccacatc 1440
   cagagcatcc tcaacccgcc aggcaacccc aaggcgcgct tcatgaccgg tgccgatcgc 1500
   ccgggtgacc cggtggcctg gcaggaaaac gccaccaagc atgccgactc ctggtggctg 1560
   cactggcaaa gctggctggg cgagcgtgcc ggcgagctgg aaaaggcgcc gacccgcctg 1620
   ggcaaccgtg cctatgccgc tggcgaggca tccccgggca cctacgttca cgagcgtgga 1680
   ttcatgaggc cagaaatcgc tgtacttgat atccaaggtc agtatcgggt ttacacggag 1740
   ttctatcgcg cggatgcggc cgaaaacacg atcatcctga tcaacggctc gctggccacc 1800
   acggcctcgt tcgcccagac ggtacgtaac ctgcacccac agttcaacgt ggttctgttc 1860
   gaccagccgt attcaggcaa gtccaagccg cacaaccgtc aggaacggct gatcagcaag 1920
   gagaccgagg cgcatatcct ccttgagctg atcgagcact tccaggcaga ccacgtgatg 1980
   tctttttcgt ggggtggcgc aagcacgctg ctggcgctgg cgcaccagcc gcggtacgtg 2040
   aagaaggcag tggtgagttc gttctcgcca gtgatcaacg agccgatgcg cgactatctg 2100
   gaccgtggct gccagtacct ggccgcctgc gaccgttatc aggtcggcaa cctggtcaat 2160
   gacaccatcg gcaagcactt gccgtcgctg ttcaaacgct tcaactaccg ccatgtgagc 2220
   agcctggaca gccacgagta cgcacagatg cacttccaca tcaaccaggt gctggagcac 2280
   gacctggaac gtgcgctgca aggcgcgcgc aatatcaaca tcccggtgct gttcatcaac 2340
   ggcgagcgcg acgagtacac cacagtcgag gatgcgcggc agttcagcaa gcatgtgggc 2400
   agaagccagt tcagcgtgat ccgcgatgcg ggccacttcc tggacatgga gaacaagacc 2460
   gcctgcgaga acacccgcaa tgtcatgctg ggcttcctca agccaaccgt gcgtgaaccc 2520
   cgccaacgtt accaacccgt gcagcagggg cagcatgcat ttgccatctg a 2571
<210> 33
   <211> 856
   <212> PRT
   <213> Artificial Sequence
<220>
   <223> Description of Artificial Sequence: Synthase Acyl ACP-CoA Transferase Fusion Protein
<220>
   <221> PEPTIDE
   <222> (1)..(856)
<400> 33
   Met Ser Asn Lys Asn Asn Asp Glu Leu Gln Arg Gln Ala Ser Glu Asn
    1               5                  10                  15
   Thr Leu Gly Leu Asn Pro Val Ile Gly Ile Arg Arg Lys Asp Leu Leu
               20                  25                  30
   Ser Ser Ala Arg Thr Val Leu Arg Gln Ala Val Arg Gln Pro Leu His
           35                  40                  45
   Ser Ala Lys His Val Ala His Phe Gly Leu Glu Leu Lys Asn Val Leu
       50                  55                  60
   Leu Gly Lys Ser Ser Leu Ala Pro Glu Ser Asp Asp Arg Arg Phe Asn
   65                  70                  75                  80
   Asp Pro Ala Trp Ser Asn Asn Pro Leu Tyr Arg Arg Tyr Leu Gln Thr
                   85                  90                  95
   Tyr Leu Ala Trp Arg Lys Glu Leu Gln Asp Trp Ile Gly Asn Ser Asp
               100                 105                 110
   Leu Ser Pro Gln Asp Ile Ser Arg Gly Gln Phe Val Ile Asn Leu Met
           115                 120                 125
   Thr Glu Ala Met Ala Pro Thr Asn Thr Leu Ser Asn Pro Ala Ala Val
       130                 135                 140
   Lys Arg Phe Phe Glu Thr Gly Gly Lys Ser Leu Leu Asp Gly Leu Ser
   145                 150                 155                 160
   Asn Leu Ala Lys Asp Leu Val Asn Asn Gly Gly Met Pro Ser Gln Val
   165 170 175
   Asn Met Asp Ala Phe Glu Val Gly Lys Asn Leu Gly Thr Ser Glu Gly
   180 185 190
   Ala Val Val Tyr Arg Asn Asp Val Leu Glu Leu Ile Gln Tyr Lys Pro
   195 200 205
   Ile Thr Glu Gln Val His Ala Arg Pro Leu Leu Val Val Pro Pro Gln
   210 215 220
   Ile Asn Lys Phe Tyr Val Phe Asp Leu Ser Pro Glu Lys Ser Leu Ala
   225 230 235 240
   Arg Tyr Cys Leu Arg Ser Gln Gln Gln Thr Phe Ile Ile Ser Trp Arg
   245 250 255
   Asn Pro Thr Lys Ala Gln Arg Glu Trp Gly Leu Ser Thr Tyr Ile Asp
   260 265 270
   Ala Leu Lys Glu Ala Val Asp Ala Val Leu Ala Ile Thr Gly Ser Lys
   275 280 285
   Asp Leu Asn Met Leu Gly Ala Cys Ser Gly Gly Ile Thr Cys Thr Ala
   290 295 300
   Leu Val Gly His Tyr Ala Ala Leu Gly Glu Asn Lys Val Asn Ala Leu
   305 310 315 320
   Thr Leu Leu Val Ser Val Leu Asp Thr Thr Met Asp Asn Gln Val Ala
   325 330 335
   Leu Phe Val Asp Glu Gln Thr Leu Glu Ala Ala Lys Arg His Ser Tyr
   340 345 350
   Gln Ala Gly Val Leu Glu Gly Ser Glu Met Ala Lys Val Phe Ala Trp
   355 360 365
   Met Arg Pro Asn Asp Leu Ile Trp Asn Tyr Trp Val Asn Asn Tyr Leu
   370 375 380
   Leu Gly Asn Glu Pro Pro Val Phe Asp Ile Leu Phe Trp Asn Asn Asp
   385 390 395 400
   Thr Thr Arg Leu Pro Ala Ala Phe His Gly Asp Leu Ile Glu Met Phe
   405 410 415
   Lys Ser Asn Pro Leu Thr Arg Pro Asp Ala Leu Glu Val Cys Gly Thr
   420 425 430
   Pro Ile Asp Leu Lys Gln Val Lys Cys Asp Ile Tyr Ser Leu Ala Gly
   435 440 445
   Thr Asn Asp His Ile Thr Pro Trp Gln Ser Cys Tyr Arg Ser Ala His
   450 455 460
   Leu Phe Gly Gly Lys Ile Glu Phe Val Leu Ser Asn Ser Gly His Ile
   465 470 475 480
   Gln Ser Ile Leu Asn Pro Pro Gly Asn Pro Lys Ala Arg Phe Met Thr
   485 490 495
   Gly Ala Asp Arg Pro Gly Asp Pro Val Ala Trp Gln Glu Asn Ala Thr
   500 505 510
   Lys His Ala Asp Ser Trp Trp Leu His Trp Gln Ser Trp Leu Gly Glu
   515 520 525
   Arg Ala Gly Glu Leu Glu Lys Ala Pro Thr Arg Leu Gly Asn Arg Ala
   530 535 540
   Tyr Ala Ala Gly Glu Ala Ser Pro Gly Thr Tyr Val His Glu Arg Gly
   545 550 555 560
   Phe Met Arg Pro Glu Ile Ala Val Leu Asp Ile Gln Gly Gln Tyr Arg
   565 570 575
   Val Tyr Thr Glu Phe Tyr Arg Ala Asp Ala Ala Glu Asn Thr Ile Ile
   580 585 590
   Leu Ile Asn Gly Ser Leu Ala Thr Thr Ala Ser Phe Ala Gln Thr Val
   595 600 605
   Arg Asn Leu His Pro Gln Phe Asn Val Val Leu Phe Asp Gln Pro Tyr
   610 615 620
   Ser Gly Lys Ser Lys Pro His Asn Arg Gln Glu Arg Leu Ile Ser Lys
   625 630 635 640
   Glu Thr Glu Ala His Ile Leu Leu Glu Leu Ile Glu His Phe Gln Ala
   645 650 655
   Asp His Val Met Ser Phe Ser Trp Gly Gly Ala Ser Thr Leu Leu Ala
   660 665 670
   Leu Ala His Gln Pro Arg Tyr Val Lys Lys Ala Val Val Ser Ser Phe
   675 680 685
   Ser Pro Val Ile Asn Glu Pro Met Arg Asp Tyr Leu Asp Arg Gly Cys
   690 695 700
   Gln Tyr Leu Ala Ala Cys Asp Arg Tyr Gln Val Gly Asn Leu Val Asn
   705 710 715 720
   Asp Thr Ile Gly Lys His Leu Pro Ser Leu Phe Lys Arg Phe Asn Tyr
   725 730 735
   Arg His Val Ser Ser Leu Asp Ser His Glu Tyr Ala Gln Met His Phe
   740 745 750
   His Ile Asn Gln Val Leu Glu His Asp Leu Glu Arg Ala Leu Gln Gly
   755 760 765
   Ala Arg Asn Ile Asn Ile Pro Val Leu Phe Ile Asn Gly Glu Arg Asp
   770 775 780
   Glu Tyr Thr Thr Val Glu Asp Ala Arg Gln Phe Ser Lys His Val Gly
   785 790 795 800
   Arg Ser Gln Phe Ser Val Ile Arg Asp Ala Gly His Phe Leu Asp Met
   805 810 815
   Glu Asn Lys Thr Ala Cys Glu Asn Thr Arg Asn Val Met Leu Gly Phe
   820 825 830
   Leu Lys Pro Thr Val Arg Glu Pro Arg Gln Arg Tyr Gln Pro Val Gln
   835 840 845
   Gln Gly Gln His Ala Phe Ala Ile
   850 855
<210> 34
   <211> 2571
   <212> DNA
   <213> Pseudomonas putida
<220>
   <221> gene
   <222> (1)..(2571)
   <223> phaG-linker-phaCl fusion gene
<400> 34
   atgaggccag aaatcgctgt acttgatatc caaggtcagt atcgggttta cacggagttc 60
   tatcgcgcgg atgcggccga aaacacgatc atcctgatca acggctcgct ggccaccacg 120
   gcctcgttcg cccagacggt acgtaacctg cacccacagt tcaacgtggt tctgttcgac 180
   cagccgtatt caggcaagtc caagccgcac aaccgtcagg aacggctgat cagcaaggag 240
   accgaggcgc atatcctcct tgagctgatc gagcacttcc aggcagacca cgtgatgtct 300
   ttttcgtggg gtggcgcaag cacgctgctg gcgctggcgc accagccgcg gtacgtgaag 360
   aaggcagtgg tgagttcgtt ctcgccagtg atcaacgagc cgatgcgcga ctatctggac 420
   cgtggctgcc agtacctggc cgcctgcgac cgttatcagg tcggcaacct ggtcaatgac 480
   accatcggca agcacttgcc gtcgctgttc aaacgcttca actaccgcca tgtgagcagc 540
   ctggacagcc acgagtacgc acagatgcac ttccacatca accaggtgct ggagcacgac 600
   ctggaacgtg cgctgcaagg cgcgcgcaat atcaacatcc cggtgctgtt catcaacggc 660
   gagcgcgacg agtacaccac agtcgaggat gcgcggcagt tcagcaagca tgtgggcaga 720
   agccagttca gcgtgatccg cgatgcgggc cacttcctgg acatggagaa caagaccgcc 780
   tgcgagaaca cccgcaatgt catgctgggc ttcctcaagc caaccgtgcg tgaaccccgc 840
   caacgttacc aacccgtgca gcaggggcag catgcatttg ccatcggatc catgagtaac 900
   aagaacaacg atgagctgca gcggcaggcc tcggaaaaca ccctggggct gaacccggtc 960
   atcggtatcc gccgcaaaga cctgttgagc tcggcacgca ccgtgctgcg ccaggccgtg 1020
   cgccaaccgc tgcacagcgc caagcatgtg gcccactttg gcctggagct gaagaacgtg 1080
   ctgctgggca agtccagcct tgccccggaa agcgacgacc gtcgcttcaa tgacccggca 1140
   tggagcaaca acccacttta ccgccgctac ctgcaaacct atctggcctg gcgcaaggag 1200
   ctgcaggact ggatcggcaa cagcgacctg tcgccccagg acatcagccg cggccagttc 1260
   gtcatcaacc tgatgaccga agccatggct ccgaccaaca ccctgtccaa cccggcagca 1320
   gtcaaacgct tcttcgaaac cggcggcaag agcctgctcg atggcctgtc caacctggcc 1380
   aaggacctgg tcaacaacgg tggcatgccc agccaggtga acatggacgc cttcgaggtg 1440
   ggcaagaacc tgggcaccag tgaaggcgcc gtggtgtacc gcaacgatgt gctggagctg 1500
   atccagtaca agcccatcac cgagcaggtg catgcccgcc cgctgctggt ggtgccgccg 1560
   cagatcaaca agttctacgt attcgacctg agcccggaaa agagcctggc acgctactgc 1620
   ctgcgctcgc agcagcagac cttcatcatc agctggcgca acccgaccaa agcccagcgc 1680
   gaatggggcc tgtccaccta catcgacgcg ctcaaggagg cggtcgacgc ggtgctggcg 1740
   attaccggca gcaaggacct gaacatgctc ggtgcctgct ccggcggcat cacctgcacg 1800
   gcattggtcg gccactatgc cgccctcggc gaaaacaagg tcaatgccct gaccctgctg 1860
   gtcagcgtgc tggacaccac catggacaac caggtcgccc tgttcgtcga cgagcagact 1920
   ttggaggccg ccaagcgcca ctcctaccag gccggtgtgc tcgaaggcag cgagatggcc 1980
   aaggtgttcg cctggatgcg ccccaacgac ctgatctgga actactgggt caacaactac 2040
   ctgctcggca acgagccgcc ggtgttcgac atcctgttct ggaacaacga caccacgcgc 2100
   ctgccggccg ccttccacgg cgacctgatc gaaatgttca agagcaaccc gctgacccgc 2160
   ccggacgccc tggaggtttg cggcactccg atcgacctga aacaggtcaa atgcgacatc 2220
   tacagccttg ccggcaccaa cgaccacatc accccgtggc agtcatgcta ccgctcggcg 2280
   cacctgttcg gcggcaagat cgagttcgtg ctgtccaaca gcggccacat ccagagcatc 2340
   ctcaacccgc caggcaaccc caaggcgcgc ttcatgaccg gtgccgatcg cccgggtgac 2400
   ccggtggcct ggcaggaaaa cgccaccaag catgccgact cctggtggct gcactggcaa 2460
   agctggctgg gcgagcgtgc cggcgagctg gaaaaggcgc cgacccgcct gggcaaccgt 2520
   gcctatgccg ctggcgaggc atccccgggc acctacgttc acgagcgttg a          2571
<210> 35
   <211> 856
   <212> PRT
   <213> Artificial Sequence
<220>
   <223> Description of Artificial Sequence: Acyl ACP-CoA Transferase Synthase Fusion Protein
<220>
   <221> PEPTIDE
   <222> (1)..(856)
<400> 35
   Met Arg Pro Glu Ile Ala Val Leu Asp Ile Gln Gly Gln Tyr Arg Val
   1 5 10 15
   Tyr Thr Glu Phe Tyr Arg Ala Asp Ala Ala Glu Asn Thr Ile Ile Leu
   20 25 30
   Ile Asn Gly Ser Leu Ala Thr Thr Ala Ser Phe Ala Gln Thr Val Arg
   35 40 45
   Asn Leu His Pro Gln Phe Asn Val Val Leu Phe Asp Gln Pro Tyr Ser
   50 55 60
   Gly Lys Ser Lys Pro His Asn Arg Gln Glu Arg Leu Ile Ser Lys Glu
   65 70 75 80
   Thr Glu Ala His Ile Leu Leu Glu Leu Ile Glu His Phe Gln Ala Asp
   85 90 95
   His Val Met Ser Phe Ser Trp Gly Gly Ala Ser Thr Leu Leu Ala Leu
   100 105 110
   Ala His Gln Pro Arg Tyr Val Lys Lys Ala Val Val Ser Ser Phe Ser
   115 120 125
   Pro Val Ile Asn Glu Pro Met Arg Asp Tyr Leu Asp Arg Gly Cys Gln
   130 135 140
   Tyr Leu Ala Ala Cys Asp Arg Tyr Gln Val Gly Asn Leu Val Asn Asp
   145 150 155 160
   Thr Ile Gly Lys His Leu Pro Ser Leu Phe Lys Arg Phe Asn Tyr Arg
   165 170 175
   His Val Ser Ser Leu Asp Ser His Glu Tyr Ala Gln Met His Phe His
   180 185 190
   Ile Asn Gln Val Leu Glu His Asp Leu Glu Arg Ala Leu Gln Gly Ala
   195 200 205
   Arg Asn Ile Asn Ile Pro Val Leu Phe Ile Asn Gly Glu Arg Asp Glu
   210 215 220
   Tyr Thr Thr Val Glu Asp Ala Arg Gln Phe Ser Lys His Val Gly Arg
   225 230 235 240
   Ser Gln Phe Ser Val Ile Arg Asp Ala Gly His Phe Leu Asp Met Glu
   245 250 255
   Asn Lys Thr Ala Cys Glu Asn Thr Arg Asn Val Met Leu Gly Phe Leu
   260 265 270
   Lys Pro Thr Val Arg Glu Pro Arg Gln Arg Tyr Gln Pro Val Gln Gln
   275 280 285
   Gly Gln His Ala Phe Ala Ile Gly Ser Met Ser Asn Lys Asn Asn Asp
   290 295 300
   Glu Leu Gln Arg Gln Ala Ser Glu Asn Thr Leu Gly Leu Asn Pro Val
   305 310 315 320
   Ile Gly Ile Arg Arg Lys Asp Leu Leu Ser Ser Ala Arg Thr Val Leu
   325 330 335
   Arg Gln Ala Val Arg Gln Pro Leu His Ser Ala Lys His Val Ala His
   340 345 350
   Phe Gly Leu Glu Leu Lys Asn Val Leu Leu Gly Lys Ser Ser Leu Ala
   355 360 365
   Pro Glu Ser Asp Asp Arg Arg Phe Asn Asp Pro Ala Trp Ser Asn Asn
   370 375 380
   Pro Leu Tyr Arg Arg Tyr Leu Gln Thr Tyr Leu Ala Trp Arg Lys Glu
   385 390 395 400
   Leu Gln Asp Trp Ile Gly Asn Ser Asp Leu Ser Pro Gln Asp Ile Ser
   405 410 415
   Arg Gly Gln Phe Val Ile Asn Leu Met Thr Glu Ala Met Ala Pro Thr
   420 425 430
   Asn Thr Leu Ser Asn Pro Ala Ala Val Lys Arg Phe Phe Glu Thr Gly
   435 440 445
   Gly Lys Ser Leu Leu Asp Gly Leu Ser Asn Leu Ala Lys Asp Leu Val
   450 455 460
   Asn Asn Gly Gly Met Pro Ser Gln Val Asn Met Asp Ala Phe Glu Val
   465 470 475 480
   Gly Lys Asn Leu Gly Thr Ser Glu Gly Ala Val Val Tyr Arg Asn Asp
   485 490 495
   Val Leu Glu Leu Ile Gln Tyr Lys Pro Ile Thr Glu Gln Val His Ala
   500 505 510
   Arg Pro Leu Leu Val Val Pro Pro Gln Ile Asn Lys Phe Tyr Val Phe
   515 520 525
   Asp Leu Ser Pro Glu Lys Ser Leu Ala Arg Tyr Cys Leu Arg Ser Gln
   530 535 540
   Gln Gln Thr Phe Ile Ile Ser Trp Arg Asn Pro Thr Lys Ala Gln Arg
   545 550 555 560
   Glu Trp Gly Leu Ser Thr Tyr Ile Asp Ala Leu Lys Glu Ala Val Asp
   565 570 575
   Ala Val Leu Ala Ile Thr Gly Ser Lys Asp Leu Asn Met Leu Gly Ala
   580 585 590
   Cys Ser Gly Gly Ile Thr Cys Thr Ala Leu Val Gly His Tyr Ala Ala
   595 600 605
   Leu Gly Glu Asn Lys Val Asn Ala Leu Thr Leu Leu Val Ser Val Leu
   610 615 620
   Asp Thr Thr Met Asp Asn Gln Val Ala Leu Phe Val Asp Glu Gln Thr
   625 630 635 640
   Leu Glu Ala Ala Lys Arg His Ser Tyr Gln Ala Gly Val Leu Glu Gly
   645 650 655
   Ser Glu Met Ala Lys Val Phe Ala Trp Met Arg Pro Asn Asp Leu Ile
   660 665 670
   Trp Asn Tyr Trp Val Asn Asn Tyr Leu Leu Gly Asn Glu Pro Pro Val
   675 680 685
   Phe Asp Ile Leu Phe Trp Asn Asn Asp Thr Thr Arg Leu Pro Ala Ala
   690 695 700
   Phe His Gly Asp Leu Ile Glu Met Phe Lys Ser Asn Pro Leu Thr Arg
   705 710 715 720
   Pro Asp Ala Leu Glu Val Cys Gly Thr Pro Ile Asp Leu Lys Gln Val
   725 730 735
   Lys Cys Asp Ile Tyr Ser Leu Ala Gly Thr Asn Asp His Ile Thr Pro
   740 745 750
   Trp Gln Ser Cys Tyr Arg Ser Ala His Leu Phe Gly Gly Lys Ile Glu
   755 760 765
   Phe Val Leu Ser Asn Ser Gly His Ile Gln Ser Ile Leu Asn Pro Pro
   770 775 780
   Gly Asn Pro Lys Ala Arg Phe Met Thr Gly Ala Asp Arg Pro Gly Asp
   785 790 795 800
   Pro Val Ala Trp Gln Glu Asn Ala Thr Lys His Ala Asp Ser Trp Trp
   805 810 815
   Leu His Trp Gln Ser Trp Leu Gly Glu Arg Ala Gly Glu Leu Glu Lys
   820 825 830
   Ala Pro Thr Arg Leu Gly Asn Arg Ala Tyr Ala Ala Gly Glu Ala Ser
   835 840 845
   Pro Gly Thr Tyr Val His Glu Arg
   850 855
<210> 36
   <211> 1731
   <212> DNA
   <213> Zoogloea ramigera
<220>
   <221> gene
   <222> (1)..(1731)
   <223> phbC gene
<400> 36
   atgaatttgc ccgatccgca agccattgcc aacgcctgga tgtcccaggt gggcgacccc 60
   agccaatggc aatcctggtt cagcaaggcg cccaccaccg aggcgaaccc gatggccacc 120
   atgttgcagg atatcggcgt tgcgctcaaa ccggaagcga tggagcagct gaaaaacgat 180
   tatctgcgtg acttcaccgc gttgtggcag gattttttgg ctggcaaggc gccagccgtc 240
   cagcgaccgc gcttcagctc ggcagcctgg cagggcaatc cgatgtcggc cttcaatgcc 300
   gcatcttacc tgctcaacgc caaattcctc agtgccatgg tggaggcggt ggacaccgca 360
   ccccagcaaa agcagaaaat acgctttgcc gtgcagcagg tgattgatgc catgtcgccc 420
   gcgaacttcc tcgccaccaa cccggaagcg cagcaaaaac tgattgaaac caagggcgag 480
   agcctgacgc gtggcctggt caatatgctg ggcgatatca atatgctggg cgatatcaac 540
   aacggccata tctcgctgtc ggacgaatcg gcctttgaag tgggccgcaa cctggccatt 600
   accccgggca ccgtgattta cgaaaatccg ctgttccagc tgatccagta cacgccgacc 660
   acgccgacgg tcagccagcg cccgctgttg atggtgccgc cgtgcatcaa caagttctac 720
   atcctcgacc tgcaaccgga aaattcgctg gtgcgctacg cggtggagca gggcaacacc 780
   gtgttcctga tctcgtggag caatccggac aagtcgctgg ccggcaccac ctgggacgac 840
   tacgtggagc agggcgtgat cgaagcgatc cgcatcgtcc aggacgtcag cggccaggac 900
   aagctgaaca tgttcggctt ctgcgtgggc ggcaccatcg ttgccaccgc actggcggta 960
   ctggcggcgc gtggccagca cccggcggcc agcctgaccc tgctgaccac cttcctcgac 1020
   ttcagcgaca ccgggtgctc gacgtcttgt cgagaaaccc aggtcgcgct gcgtgaacag 1080
   caattgcgcg atggcggcct gatgccgggc cgtgacctgg cctcgacctt ctcgagcctg 1140
   cgtccgaacg acctggtatg gaactatgtg cagtcgaact acctcaaagg caatgagccg 1200
   gcggcgtttg acctgctgtt ctggaattcg gacagcacca atttgccggg cccgatgttc 1260
   tgctggtacc tgcgcaacac ctacctggaa aacagcctga aagtgccggg caagctgacg 1320
   gtggccggcg aaaagatcga cctcggcctg atcgacgccc cggccttcat ctacggttcg 1380
   cgcgaagacc acatcgtgcc gtggatgtcg gcgtacggtt cgctcgacat cctgaaccag 1440
   ggcaagccgg gcgccaaccg cttcgtgctg ggcgcgtccg gccatatcgc cggcgtgatc 1500
   aactcggtgg ccaagaacaa gcgcacgtac tggatcaacg acggtggcgc cgccgatgcc 1560
   caggcctggt tcgatggcgc gcaggaagtg ccgggcagct ggtggccgca atgggccggg 1620
   ttcctgaccc agcatggcgg caagaaggtc aagcccaagg ccaagcccgg caacgcccgc 1680
   tacaccgcga tcgaggcggc gcccggccgt tacgtcaaag ccaagggctg a          1731
<210> 37
   <211> 576
   <212> PRT
   <213> Zoogloea ramigera
<220>
   <221> PEPTIDE
   <222> (1)..(576)
<400> 37
   Met Asn Leu Pro Asp Pro Gln Ala Ile Ala Asn Ala Trp Met Ser Gln
   1               5                   10                  15
   Val Gly Asp Pro Ser Gln Trp Gln Ser Trp Phe Ser Lys Ala Pro Thr
               20                  25                  30
   Thr Glu Ala Asn Pro Met Ala Thr Met Leu Gln Asp Ile Gly Val Ala
           35                  40                  45
   Leu Lys Pro Glu Ala Met Glu Gln Leu Lys Asn Asp Tyr Leu Arg Asp
       50                  55                  60
   Phe Thr Ala Leu Trp Gln Asp Phe Leu Ala Gly Lys Ala Pro Ala Val
   65 70 75 80
   Gln Arg Pro Arg Phe Ser Ser Ala Ala Trp Gln Gly Asn Pro Met Ser
   85 90 95
   Ala Phe Asn Ala Ala Ser Tyr Leu Leu Asn Ala Lys Phe Leu Ser Ala
   100 105 110
   Met Val Glu Ala Val Asp Thr Ala Pro Gln Gln Lys Gln Lys Ile Arg
   115 120 125
   Phe Ala Val Gln Gln Val Ile Asp Ala Met Ser Pro Ala Asn Phe Leu
   130 135 140
   Ala Thr Asn Pro Glu Ala Gln Gln Lys Leu Ile Glu Thr Lys Gly Glu
   145 150 155 160
   Ser Leu Thr Arg Gly Leu Val Asn Met Leu Gly Asp Ile Asn Met Leu
   165 170 175
   Gly Asp Ile Asn Asn Gly His Ile Ser Leu Ser Asp Glu Ser Ala Phe
   180 185 190
   Glu Val Gly Arg Asn Leu Ala Ile Thr Pro Gly Thr Val Ile Tyr Glu
   195 200 205
   Asn Pro Leu Phe Gln Leu Ile Gln Tyr Thr Pro Thr Thr Pro Thr Val
   210 215 220
   Ser Gln Arg Pro Leu Leu Met Val Pro Pro Cys Ile Asn Lys Phe Tyr
   225 230 235 240
   Ile Leu Asp Leu Gln Pro Glu Asn Ser Leu Val Arg Tyr Ala Val Glu
   245 250 255
   Gln Gly Asn Thr Val Phe Leu Ile Ser Trp Ser Asn Pro Asp Lys Ser
   260 265 270
   Leu Ala Gly Thr Thr Trp Asp Asp Tyr Val Glu Gln Gly Val Ile Glu
   275 280 285
   Ala Ile Arg Ile Val Gln Asp Val Ser Gly Gln Asp Lys Leu Asn Met
   290 295 300
   Phe Gly Phe Cys Val Gly Gly Thr Ile Val Ala Thr Ala Leu Ala Val
   305 310 315 320
   Leu Ala Ala Arg Gly Gln His Pro Ala Ala Ser Leu Thr Leu Leu Thr
   325 330 335
   Thr Phe Leu Asp Phe Ser Asp Thr Gly Cys Ser Thr Ser Cys Arg Glu
   340 345 350
   Thr Gln Val Ala Leu Arg Glu Gln Gln Leu Arg Asp Gly Gly Leu Met
   355 360 365
   Pro Gly Arg Asp Leu Ala Ser Thr Phe Ser Ser Leu Arg Pro Asn Asp
   370 375 380
   Leu Val Trp Asn Tyr Val Gln Ser Asn Tyr Leu Lys Gly Asn Glu Pro
   385 390 395 400
   Ala Ala Phe Asp Leu Leu Phe Trp Asn Ser Asp Ser Thr Asn Leu Pro
   405 410 415
   Gly Pro Met Phe Cys Trp Tyr Leu Arg Asn Thr Tyr Leu Glu Asn Ser
   420 425 430
   Leu Lys Val Pro Gly Lys Leu Thr Val Ala Gly Glu Lys Ile Asp Leu
   435 440 445
   Gly Leu Ile Asp Ala Pro Ala Phe Ile Tyr Gly Ser Arg Glu Asp His
   450 455 460
   Ile Val Pro Trp Met Ser Ala Tyr Gly Ser Leu Asp Ile Leu Asn Gln
   465 470 475 480
   Gly Lys Pro Gly Ala Asn Arg Phe Val Leu Gly Ala Ser Gly His Ile
   485 490 495
   Ala Gly Val Ile Asn Ser Val Ala Lys Asn Lys Arg Thr Tyr Trp Ile
   500 505 510
   Asn Asp Gly Gly Ala Ala Asp Ala Gln Ala Trp Phe Asp Gly Ala Gln
   515 520 525
   Glu Val Pro Gly Ser Trp Trp Pro Gln Trp Ala Gly Phe Leu Thr Gln
   530 535 540
   His Gly Gly Lys Lys Val Lys Pro Lys Ala Lys Pro Gly Asn Ala Arg
   545 550 555 560
   Tyr Thr Ala Ile Glu Ala Ala Pro Gly Arg Tyr Val Lys Ala Lys Gly
   565 570 575
<210> 38
   <211> 42
   <212> DNA
   <213> Artificial Sequence
<220>
   <223> Description of Artificial Sequence: oligonucleotide primer- C5 up I
<400> 38
   ggagctcagg aggttttatg agtaacaaga acaacgatga gc 42
<210> 39
   <211> 30
   <212> DNA
   <213> Artificial Sequence
<220>
   <223> Description of Artificial Sequence: oligonucleotide primer-C5 up II
<400> 39
   cgggatccgc ccttggcttt gacgtaacgg 30
<210> 40
   <211> 30
   <212> DNA
   <213> Artificial Sequence
<220>
   <223> Description of Artificial Sequence: oligonucleotide primer- C5 dw I
<400> 40
   cgggatccag taacaagaac aacgatgagc 30
<210> 41
   <211> 38
   <212> DNA
   <213> Artificial Sequence
<220>
   <223> Description of Artificial Sequence: oligonucleotide primer- C5 dw II
<400> 41
   gctctagaag ctttcagccc ttggctttga cgtaacgg 38
<210> 42
   <211> 405
   <212> DNA
   <213> Aeromonas caviae
<220>
   <221> gene
   <222> (1)..(405)
   <223> phbJ gene
<400> 42
   atgagcgcac aatccctgga agtaggccag aaggcccgtc tcagcaagcg gttcggggcg 60
   gcggaggtag ccgccttcgc cgcgctctcg gaggacttca accccctgca cctggacccg 120
   gccttcgccg ccaccacggc gttcgagcgg cccatagtcc acggcatgct gctcgccagc 180
   ctcttctccg ggctgctggg ccagcagttg ccgggcaagg ggagcatcta tctgggtcaa 240
   agcctcagct tcaagctgcc ggtctttgtc ggggacgagg tgacggccga ggtggaggtg 300
   accgcccttc gcgaggacaa gcccatcgcc accctgacca cccgcatctt cacccaaggc 360
   ggcgccctcg ccgtgacggg ggaagccgtg gtcaagctgc cttaa                405
<210> 43
   <211> 134
   <212> PRT
   <213> Artificial Sequence
<220>
   <223> Description of Artificial Sequence: (R) specific enoyl-CoA transferase
<220>
   <221> PEPTIDE
   <222> (1)..(134)
<400> 43
   Met Ser Ala Gln Ser Leu Glu Val Gly Gln Lys Ala Arg Leu Ser Lys
   1               5                   10                  15
   Arg Phe Gly Ala Ala Glu Val Ala Ala Phe Ala Ala Leu Ser Glu Asp
               20                  25                  30
   Phe Asn Pro Leu His Leu Asp Pro Ala Phe Ala Ala Thr Thr Ala Phe
   35 40 45
   Glu Arg Pro Ile Val His Gly Met Leu Leu Ala Ser Leu Phe Ser Gly
   50 55 60
   Leu Leu Gly Gln Gln Leu Pro Gly Lys Gly Ser Ile Tyr Leu Gly Gln
   65 70 75 80
   Ser Leu Ser Phe Lys Leu Pro Val Phe Val Gly Asp Glu Val Thr Ala
   85 90 95
   Glu Val Glu Val Thr Ala Leu Arg Glu Asp Lys Pro Ile Ala Thr Leu
   100 105 110
   Thr Thr Arg Ile Phe Thr Gln Gly Gly Ala Leu Ala Val Thr Gly Glu
   115 120 125
   Ala Val Val Lys Leu Pro
   130
<210> 44
   <211> 30
   <212> DNA
   <213> Artificial Sequence
<220>
   <223> Description of Artificial Sequence: oligonucleotide primer- J12 dw I
<400> 44
   cgggatccag cgcacaatcc ctggaagtag 30
<210> 45
   <211> 38
   <212> DNA
   <213> Artificial Sequence
<220>
   <223> Description of Artificial Sequence: oligonucleotide primer-J12 dw III
<400> 45
   gctctagaag cttttaaggc agcttgacca cggcttcc 38
<210> 46
   <211> 43
   <212> DNA
   <213> Artificial Sequence
<220>
   <223> Description of Artificial Sequence: J12 up I
<400> 46
   aggagctcag gaggttttat gagcgcacaa tccctggaag tag 43
<210> 47
   <211> 30
   <212> DNA
   <213> Artificial Sequence
<220>
   <223> Description of Artificial Sequence: oligonucleotide primer- J12 up III
<400> 47
   cgggatccag gcagcttgac cacggcttcc 30
<210> 48
   <211> 2139
   <212> DNA
   <213> Aeromonas caviae
<220>
   <221> gene
   <222> (1)..(2139)
   <223> phaC-linker-phbJ fusion gene
<400> 48
   atgaatttgc ccgatccgca agccattgcc aacgcctgga tgtcccaggt gggcgacccc 60
   agccaatggc aatcctggtt cagcaaggcg cccaccaccg aggcgaaccc gatggccacc 120
   atgttgcagg atatcggcgt tgcgctcaaa ccggaagcga tggagcagct gaaaaacgat 180
   tatctgcgtg acttcaccgc gttgtggcag gattttttgg ctggcaaggc gccagccgtc 240
   cagcgaccgc gcttcagctc ggcagcctgg cagggcaatc cgatgtcggc cttcaatgcc 300
   gcatcttacc tgctcaacgc caaattcctc agtgccatgg tggaggcggt ggacaccgca 360
   ccccagcaaa agcagaaaat acgctttgcc gtgcagcagg tgattgatgc catgtcgccc 420
   gcgaacttcc tcgccaccaa cccggaagcg cagcaaaaac tgattgaaac caagggcgag 480
   agcctgacgc gtggcctggt caatatgctg ggcgatatca atatgctggg cgatatcaac 540
   aacggccata tctcgctgtc ggacgaatcg gcctttgaag tgggccgcaa cctggccatt 600
   accccgggca ccgtgattta cgaaaatccg ctgttccagc tgatccagta cacgccgacc 660
   acgccgacgg tcagccagcg cccgctgttg atggtgccgc cgtgcatcaa caagttctac 720
   atcctcgacc tgcaaccgga aaattcgctg gtgcgctacg cggtggagca gggcaacacc 780
   gtgttcctga tctcgtggag caatccggac aagtcgctgg ccggcaccac ctgggacgac 840
   tacgtggagc agggcgtgat cgaagcgatc cgcatcgtcc aggacgtcag cggccaggac 900
   aagctgaaca tgttcggctt ctgcgtgggc ggcaccatcg ttgccaccgc actggcggta 960
   ctggcggcgc gtggccagca cccggcggcc agcctgaccc tgctgaccac cttcctcgac 1020
   ttcagcgaca ccgggtgctc gacgtcttgt cgagaaaccc aggtcgcgct gcgtgaacag 1080
   caattgcgcg atggcggcct gatgccgggc cgtgacctgg cctcgacctt ctcgagcctg 1140
   cgtccgaacg acctggtatg gaactatgtg cagtcgaact acctcaaagg caatgagccg 1200
   gcggcgtttg acctgctgtt ctggaattcg gacagcacca atttgccggg cccgatgttc 1260
   tgctggtacc tgcgcaacac ctacctggaa aacagcctga aagtgccggg caagctgacg 1320
   gtggccggcg aaaagatcga cctcggcctg atcgacgccc cggccttcat ctacggttcg 1380
   cgcgaagacc acatcgtgcc gtggatgtcg gcgtacggtt cgctcgacat cctgaaccag 1440
   ggcaagccgg gcgccaaccg cttcgtgctg ggcgcgtccg gccatatcgc cggcgtgatc 1500
   aactcggtgg ccaagaacaa gcgcacgtac tggatcaacg acggtggcgc cgccgatgcc 1560
   caggcctggt tcgatggcgc gcaggaagtg ccgggcagct ggtggccgca atgggccggg 1620
   ttcctgaccc agcatggcgg caagaaggtc aagcccaagg ccaagcccgg caacgcccgc 1680
   tacaccgcga tcgaggcggc gcccggccgt tacgtcaaag ccaagggcgg atccatgagc 1740
   gcacaatccc tggaagtagg ccagaaggcc cgtctcagca agcggttcgg ggcggcggag 1800
   gtagccgcct tcgccgcgct ctcggaggac ttcaaccccc tgcacctgga cccggccttc 1860
   gccgccacca cggcgttcga gcggcccata gtccacggca tgctgctcgc cagcctcttc 1920
   tccgggctgc tgggccagca gttgccgggc aaggggagca tctatctggg tcaaagcctc 1980
   agcttcaagc tgccggtctt tgtcggggac gaggtgacgg ccgaggtgga ggtgaccgcc 2040
   cttcgcgagg acaagcccat cgccaccctg accacccgca tcttcaccca aggcggcgcc 2100
   ctcgccgtga cgggggaagc cgtggtcaag ctgccttaa 2139
<210> 49
   <211> 712
   <212> PRT
   <213> Artificial Sequence
<220>
   <223> Description of Artificial Sequence: Synthase (R) specific enoyl-CoA transferase Fusion Protein
<220>
   <221> PEPTIDE
   <222> (1)..(712)
<400> 49
   Met Asn Leu Pro Asp Pro Gln Ala Ile Ala Asn Ala Trp Met Ser Gln
   1               5                   10                  15
   Val Gly Asp Pro Ser Gln Trp Gln Ser Trp Phe Ser Lys Ala Pro Thr
               20                  25                  30
   Thr Glu Ala Asn Pro Met Ala Thr Met Leu Gln Asp Ile Gly Val Ala
           35                  40                  45
   Leu Lys Pro Glu Ala Met Glu Gln Leu Lys Asn Asp Tyr Leu Arg Asp
   50 55 60
   Phe Thr Ala Leu Trp Gln Asp Phe Leu Ala Gly Lys Ala Pro Ala Val
   65 70 75 80
   Gln Arg Pro Arg Phe Ser Ser Ala Ala Trp Gln Gly Asn Pro Met Ser
   85 90 95
   Ala Phe Asn Ala Ala Ser Tyr Leu Leu Asn Ala Lys Phe Leu Ser Ala
   100 105 110
   Met Val Glu Ala Val Asp Thr Ala Pro Gln Gln Lys Gln Lys Ile Arg
   115 120 125
   Phe Ala Val Gln Gln Val Ile Asp Ala Met Ser Pro Ala Asn Phe Leu
   130 135 140
   Ala Thr Asn Pro Glu Ala Gln Gln Lys Leu Ile Glu Thr Lys Gly Glu
   145 150 155 160
   Ser Leu Thr Arg Gly Leu Val Asn Met Leu Gly Asp Ile Asn Met Leu
   165 170 175
   Gly Asp Ile Asn Asn Gly His Ile Ser Leu Ser Asp Glu Ser Ala Phe
   180 185 190
   Glu Val Gly Arg Asn Leu Ala Ile Thr Pro Gly Thr Val Ile Tyr Glu
   195 200 205
   Asn Pro Leu Phe Gln Leu Ile Gln Tyr Thr Pro Thr Thr Pro Thr Val
   210 215 220
   Ser Gln Arg Pro Leu Leu Met Val Pro Pro Cys Ile Asn Lys Phe Tyr
   225 230 235 240
   Ile Leu Asp Leu Gln Pro Glu Asn Ser Leu Val Arg Tyr Ala Val Glu
   245 250 255
   Gln Gly Asn Thr Val Phe Leu Ile Ser Trp Ser Asn Pro Asp Lys Ser
   260 265 270
   Leu Ala Gly Thr Thr Trp Asp Asp Tyr Val Glu Gln Gly Val Ile Glu
   275 280 285
   Ala Ile Arg Ile Val Gln Asp Val Ser Gly Gln Asp Lys Leu Asn Met
   290 295 300
   Phe Gly Phe Cys Val Gly Gly Thr Ile Val Ala Thr Ala Leu Ala Val
   305 310 315 320
   Leu Ala Ala Arg Gly Gln His Pro Ala Ala Ser Leu Thr Leu Leu Thr
   325 330 335
   Thr Phe Leu Asp Phe Ser Asp Thr Gly Cys Ser Thr Ser Cys Arg Glu
   340 345 350
   Thr Gln Val Ala Leu Arg Glu Gln Gln Leu Arg Asp Gly Gly Leu Met
   355 360 365
   Pro Gly Arg Asp Leu Ala Ser Thr Phe Ser Ser Leu Arg Pro Asn Asp
   370 375 380
   Leu Val Trp Asn Tyr Val Gln Ser Asn Tyr Leu Lys Gly Asn Glu Pro
   385 390 395 400
   Ala Ala Phe Asp Leu Leu Phe Trp Asn Ser Asp Ser Thr Asn Leu Pro
   405 410 415
   Gly Pro Met Phe Cys Trp Tyr Leu Arg Asn Thr Tyr Leu Glu Asn Ser
   420 425 430
   Leu Lys Val Pro Gly Lys Leu Thr Val Ala Gly Glu Lys Ile Asp Leu
   435 440 445
   Gly Leu Ile Asp Ala Pro Ala Phe Ile Tyr Gly Ser Arg Glu Asp His
   450 455 460
   Ile Val Pro Trp Met Ser Ala Tyr Gly Ser Leu Asp Ile Leu Asn Gln
   465 470 475 480
   Gly Lys Pro Gly Ala Asn Arg Phe Val Leu Gly Ala Ser Gly His Ile
   485 490 495
   Ala Gly Val Ile Asn Ser Val Ala Lys Asn Lys Arg Thr Tyr Trp Ile
   500 505 510
   Asn Asp Gly Gly Ala Ala Asp Ala Gln Ala Trp Phe Asp Gly Ala Gln
   515 520 525
   Glu Val Pro Gly Ser Trp Trp Pro Gln Trp Ala Gly Phe Leu Thr Gln
   530 535 540
   His Gly Gly Lys Lys Val Lys Pro Lys Ala Lys Pro Gly Asn Ala Arg
   545 550 555 560
   Tyr Thr Ala Ile Glu Ala Ala Pro Gly Arg Tyr Val Lys Ala Lys Gly
   565 570 575
   Gly Ser Met Ser Ala Gln Ser Leu Glu Val Gly Gln Lys Ala Arg Leu
   580 585 590
   Ser Lys Arg Phe Gly Ala Ala Glu Val Ala Ala Phe Ala Ala Leu Ser
   595 600 605
   Glu Asp Phe Asn Pro Leu His Leu Asp Pro Ala Phe Ala Ala Thr Thr
   610 615 620
   Ala Phe Glu Arg Pro Ile Val His Gly Met Leu Leu Ala Ser Leu Phe
   625 630 635 640
   Ser Gly Leu Leu Gly Gln Gln Leu Pro Gly Lys Gly Ser Ile Tyr Leu
   645 650 655
   Gly Gln Ser Leu Ser Phe Lys Leu Pro Val Phe Val Gly Asp Glu Val
   660 665 670
   Thr Ala Glu Val Glu Val Thr Ala Leu Arg Glu Asp Lys Pro Ile Ala
   675 680 685
   Thr Leu Thr Thr Arg Ile Phe Thr Gln Gly Gly Ala Leu Ala Val Thr
   690 695 700
   Gly Glu Ala Val Val Lys Leu Pro
   705 710
<210> 50
   <211> 2139
   <212> DNA
   <213> Zoogloea ramigera
<220>
   <221> gene
   <222> (1)..(2139)
   <223> phbJ-lonker-phaC fusion gene
<400> 50
   atgagcgcac aatccctgga agtaggccag aaggcccgtc tcagcaagcg gttcggggcg 60
   gcggaggtag ccgccttcgc cgcgctctcg gaggacttca accccctgca cctggacccg 120
   gccttcgccg ccaccacggc gttcgagcgg cccatagtcc acggcatgct gctcgccagc 180
   ctcttctccg ggctgctggg ccagcagttg ccgggcaagg ggagcatcta tctgggtcaa 240
   agcctcagct tcaagctgcc ggtctttgtc ggggacgagg tgacggccga ggtggaggtg 300
   accgcccttc gcgaggacaa gcccatcgcc accctgacca cccgcatctt cacccaaggc 360
   ggcgccctcg ccgtgacggg ggaagccgtg gtcaagctgc ctggatccat gaatttgccc 420
   gatccgcaag ccattgccaa cgcctggatg tcccaggtgg gcgaccccag ccaatggcaa 480
   tcctggttca gcaaggcgcc caccaccgag gcgaacccga tggccaccat gttgcaggat 540
   atcggcgttg cgctcaaacc ggaagcgatg gagcagctga aaaacgatta tctgcgtgac 600
   ttcaccgcgt tgtggcagga ttttttggct ggcaaggcgc cagccgtcca gcgaccgcgc 660
   ttcagctcgg cagcctggca gggcaatccg atgtcggcct tcaatgccgc atcttacctg 720
   ctcaacgcca aattcctcag tgccatggtg gaggcggtgg acaccgcacc ccagcaaaag 780
   cagaaaatac gctttgccgt gcagcaggtg attgatgcca tgtcgcccgc gaacttcctc 840
   gccaccaacc cggaagcgca gcaaaaactg attgaaacca agggcgagag cctgacgcgt 900
   ggcctggtca atatgctggg cgatatcaat atgctgggcg atatcaacaa cggccatatc 960
   tcgctgtcgg acgaatcggc ctttgaagtg ggccgcaacc tggccattac cccgggcacc 1020
   gtgatttacg aaaatccgct gttccagctg atccagtaca cgccgaccac gccgacggtc 1080
   agccagcgcc cgctgttgat ggtgccgccg tgcatcaaca agttctacat cctcgacctg 1140
   caaccggaaa attcgctggt gcgctacgcg gtggagcagg gcaacaccgt gttcctgatc 1200
   tcgtggagca atccggacaa gtcgctggcc ggcaccacct gggacgacta cgtggagcag 1260
   ggcgtgatcg aagcgatccg catcgtccag gacgtcagcg gccaggacaa gctgaacatg 1320
   ttcggcttct gcgtgggcgg caccatcgtt gccaccgcac tggcggtact ggcggcgcgt 1380
   ggccagcacc cggcggccag cctgaccctg ctgaccacct tcctcgactt cagcgacacc 1440
   gggtgctcga cgtcttgtcg agaaacccag gtcgcgctgc gtgaacagca attgcgcgat 1500
   ggcggcctga tgccgggccg tgacctggcc tcgaccttct cgagcctgcg tccgaacgac 1560
   ctggtatgga actatgtgca gtcgaactac ctcaaaggca atgagccggc ggcgtttgac 1620
   ctgctgttct ggaattcgga cagcaccaat ttgccgggcc cgatgttctg ctggtacctg 1680
   cgcaacacct acctggaaaa cagcctgaaa gtgccgggca agctgacggt ggccggcgaa 1740
   aagatcgacc tcggcctgat cgacgccccg gccttcatct acggttcgcg cgaagaccac 1800
   atcgtgccgt ggatgtcggc gtacggttcg ctcgacatcc tgaaccaggg caagccgggc 1860
   gccaaccgct tcgtgctggg cgcgtccggc catatcgccg gcgtgatcaa ctcggtggcc 1920
   aagaacaagc gcacgtactg gatcaacgac ggtggcgccg ccgatgccca ggcctggttc 1980
   gatggcgcgc aggaagtgcc gggcagctgg tggccgcaat gggccgggtt cctgacccag 2040
   catggcggca agaaggtcaa gcccaaggcc aagcccggca acgcccgcta caccgcgatc 2100
   gaggcggcgc ccggccgtta cgtcaaagcc aagggctga                        2139
<210> 51
   <211> 712
   <212> PRT
   <213> Artificial Sequence
<220>
   <223> Description of Artificial Sequence: (R) - specific enoyl-CoA transferase Synthase Fusion Protein
<220>
   <221> PEPTIDE
   <222> (1)..(712)
<400> 51
   Met Ser Ala Gln Ser Leu Glu Val Gly Gln Lys Ala Arg Leu Ser Lys
   1               5                   10                  15
   Arg Phe Gly Ala Ala Glu Val Ala Ala Phe Ala Ala Leu Ser Glu Asp
   20 25 30
   Phe Asn Pro Leu His Leu Asp Pro Ala Phe Ala Ala Thr Thr Ala Phe
   35 40 45
   Glu Arg Pro Ile Val His Gly Met Leu Leu Ala Ser Leu Phe Ser Gly
   50 55 60
   Leu Leu Gly Gln Gln Leu Pro Gly Lys Gly Ser Ile Tyr Leu Gly Gln
   65 70 75 80
   Ser Leu Ser Phe Lys Leu Pro Val Phe Val Gly Asp Glu Val Thr Ala
   85 90 95
   Glu Val Glu Val Thr Ala Leu Arg Glu Asp Lys Pro Ile Ala Thr Leu
   100 105 110
   Thr Thr Arg Ile Phe Thr Gln Gly Gly Ala Leu Ala Val Thr Gly Glu
   115 120 125
   Ala Val Val Lys Leu Pro Gly Ser Met Asn Leu Pro Asp Pro Gln Ala
   130 135 140
   Ile Ala Asn Ala Trp Met Ser Gln Val Gly Asp Pro Ser Gln Trp Gln
   145 150 155 160
   Ser Trp Phe Ser Lys Ala Pro Thr Thr Glu Ala Asn Pro Met Ala Thr
   165 170 175
   Met Leu Gln Asp Ile Gly Val Ala Leu Lys Pro Glu Ala Met Glu Gln
   180 185 190
   Leu Lys Asn Asp Tyr Leu Arg Asp Phe Thr Ala Leu Trp Gln Asp Phe
   195 200 205
   Leu Ala Gly Lys Ala Pro Ala Val Gln Arg Pro Arg Phe Ser Ser Ala
   210 215 220
   Ala Trp Gln Gly Asn Pro Met Ser Ala Phe Asn Ala Ala Ser Tyr Leu
   225 230 235 240
   Leu Asn Ala Lys Phe Leu Ser Ala Met Val Glu Ala Val Asp Thr Ala
   245 250 255
   Pro Gln Gln Lys Gln Lys Ile Arg Phe Ala Val Gln Gln Val Ile Asp
   260 265 270
   Ala Met Ser Pro Ala Asn Phe Leu Ala Thr Asn Pro Glu Ala Gln Gln
   275 280 285
   Lys Leu Ile Glu Thr Lys Gly Glu Ser Leu Thr Arg Gly Leu Val Asn
   290 295 300
   Met Leu Gly Asp Ile Asn Met Leu Gly Asp Ile Asn Asn Gly His Ile
   305 310 315 320
   Ser Leu Ser Asp Glu Ser Ala Phe Glu Val Gly Arg Asn Leu Ala Ile
   325 330 335
   Thr Pro Gly Thr Val Ile Tyr Glu Asn Pro Leu Phe Gln Leu Ile Gln
   340 345 350
   Tyr Thr Pro Thr Thr Pro Thr Val Ser Gln Arg Pro Leu Leu Met Val
   355 360 365
   Pro Pro Cys Ile Asn Lys Phe Tyr Ile Leu Asp Leu Gln Pro Glu Asn
   370 375 380
   Ser Leu Val Arg Tyr Ala Val Glu Gln Gly Asn Thr Val Phe Leu Ile
   385 390 395 400
   Ser Trp Ser Asn Pro Asp Lys Ser Leu Ala Gly Thr Thr Trp Asp Asp
   405 410 415
   Tyr Val Glu Gln Gly Val Ile Glu Ala Ile Arg Ile Val Gln Asp Val
   420 425 430
   Ser Gly Gln Asp Lys Leu Asn Met Phe Gly Phe Cys Val Gly Gly Thr
   435 440 445
   Ile Val Ala Thr Ala Leu Ala Val Leu Ala Ala Arg Gly Gln His Pro
   450 455 460
   Ala Ala Ser Leu Thr Leu Leu Thr Thr Phe Leu Asp Phe Ser Asp Thr
   465 470 475 480
   Gly Cys Ser Thr Ser Cys Arg Glu Thr Gln Val Ala Leu Arg Glu Gln
   485 490 495
   Gln Leu Arg Asp Gly Gly Leu Met Pro Gly Arg Asp Leu Ala Ser Thr
   500 505 510
   Phe Ser Ser Leu Arg Pro Asn Asp Leu Val Trp Asn Tyr Val Gln Ser
   515 520 525
   Asn Tyr Leu Lys Gly Asn Glu Pro Ala Ala Phe Asp Leu Leu Phe Trp
   530 535 540
   Asn Ser Asp Ser Thr Asn Leu Pro Gly Pro Met Phe Cys Trp Tyr Leu
   545 550 555 560
   Arg Asn Thr Tyr Leu Glu Asn Ser Leu Lys Val Pro Gly Lys Leu Thr
   565 570 575
   Val Ala Gly Glu Lys Ile Asp Leu Gly Leu Ile Asp Ala Pro Ala Phe
   580 585 590
   Ile Tyr Gly Ser Arg Glu Asp His Ile Val Pro Trp Met Ser Ala Tyr
   595 600 605
   Gly Ser Leu Asp Ile Leu Asn Gln Gly Lys Pro Gly Ala Asn Arg Phe
   610 615 620
   Val Leu Gly Ala Ser Gly His Ile Ala Gly Val Ile Asn Ser Val Ala
   625 630 635 640
   Lys Asn Lys Arg Thr Tyr Trp Ile Asn Asp Gly Gly Ala Ala Asp Ala
   645 650 655
   Gln Ala Trp Phe Asp Gly Ala Gln Glu Val Pro Gly Ser Trp Trp Pro
   660 665 670
   Gln Trp Ala Gly Phe Leu Thr Gln His Gly Gly Lys Lys Val Lys Pro
   675 680 685
   Lys Ala Lys Pro Gly Asn Ala Arg Tyr Thr Ala Ile Glu Ala Ala Pro
   690 695 700
   Gly Arg Tyr Val Lys Ala Lys Gly
   705 710
<210> 52
   <211> 1185
   <212> DNA
   <213> Aeromonas caviae
<220>
   <221> gene
   <222> (1)..(1185)
   <223> bktB gene
<400> 52
   atgacgcgtg aagtggtagt ggtaagcggt gtccgtaccg cgatcgggac ctttggcggc 60
   agcctgaagg atgtggcacc ggcggagctg ggcgcactgg tggtgcgcga ggcgctggcg 120
   cgcgcgcagg tgtcgggcga cgatgtcggc cacgtggtat tcggcaacgt gatccagacc 180
   gagccgcgcg acatgtatct gggccgcgtc gcggccgtca acggcggggt gacgatcaac 240
   gcccccgcgc tgaccgtgaa ccgcctgtgc ggctcgggcc tgcaggccat tgtcagcgcc 300
   gcgcagacca tcctgctggg cgataccgac gtcgccatcg gcggcggcgc ggaaagcatg 360
   agccgcgcac cgtacctggc gccggcagcg cgctggggcg cacgcatggg cgacgccggc 420
   ctggtcgaca tgatgctggg tgcgctgcac gatcccttcc atcgcatcca catgggcgtg 480
   accgccgaga atgtcgccaa ggaatacgac atctcgcgcg cgcagcagga cgaggccgcg 540
   ctggaatcgc accgccgcgc ttcggcagcg atcaaggccg gctacttcaa ggaccagatc 600
   gtcccggtgg tgagcaaggg ccgcaagggc gacgtgacct tcgacaccga cgagcacgtg 660
   cgccatgacg ccaccatcga cgacatgacc aagctcaggc cggtcttcgt caaggaaaac 720
   ggcacggtca cggccggcaa tgcctcgggc ctgaacgacg ccgccgccgc ggtggtgatg 780
   atggagcgcg ccgaagccga gcgccgcggc ctgaagccgc tggcccgcct ggtgtcgtac 840
   ggccatgccg gcgtggaccc gaaggccatg ggcatcggcc cggtgccggc gacgaagatc 900
   gcgctggagc gcgccggcct gcaggtgtcg gacctggacg tgatcgaagc caacgaagcc 960
   tttgccgcac aggcgtgcgc cgtgaccaag gcgctcggtc tggacccggc caaggttaac 1020
   ccgaacggct cgggcatctc gctgggccac ccgatcggcg ccaccggtgc cctgatcacg 1080
   gtgaaggcgc tgcatgagct gaaccgcgtg cagggccgct acgcgctggt gacgatgtgc 1140
   atcggcggcg ggcagggcat tgccgccatc ttcgagcgta tctga 1185
<210> 53
   <211> 394
   <212> PRT
   <213> Artificial Sequence
<220>
   <223> Description of Artificial Sequence: thiolase II
<220>
   <221> PEPTIDE
   <222> (1)..(394)
<400> 53
   Met Thr Arg Glu Val Val Val Val Ser Gly Val Arg Thr Ala Ile Gly
   1 5 10 15
   Thr Phe Gly Gly Ser Leu Lys Asp Val Ala Pro Ala Glu Leu Gly Ala
   20 25 30
   Leu Val Val Arg Glu Ala Leu Ala Arg Ala Gln Val Ser Gly Asp Asp
   35 40 45
   Val Gly His Val Val Phe Gly Asn Val Ile Gln Thr Glu Pro Arg Asp
   50 55 60
   Met Tyr Leu Gly Arg Val Ala Ala Val Asn Gly Gly Val Thr Ile Asn
   65 70 75 80
   Ala Pro Ala Leu Thr Val Asn Arg Leu Cys Gly Ser Gly Leu Gln Ala
   85 90 95
   Ile Val Ser Ala Ala Gln Thr Ile Leu Leu Gly Asp Thr Asp Val Ala
   100 105 110
   Ile Gly Gly Gly Ala Glu Ser Met Ser Arg Ala Pro Tyr Leu Ala Pro
   115 120 125
   Ala Ala Arg Trp Gly Ala Arg Met Gly Asp Ala Gly Leu Val Asp Met
   130 135 140
   Met Leu Gly Ala Leu His Asp Pro Phe His Arg Ile His Met Gly Val
   145 150 155 160
   Thr Ala Glu Asn Val Ala Lys Glu Tyr Asp Ile Ser Arg Ala Gln Gln
   165 170 175
   Asp Glu Ala Ala Leu Glu Ser His Arg Arg Ala Ser Ala Ala Ile Lys
   180 185 190
   Ala Gly Tyr Phe Lys Asp Gln Ile Val Pro Val Val Ser Lys Gly Arg
   195 200 205
   Lys Gly Asp Val Thr Phe Asp Thr Asp Glu His Val Arg His Asp Ala
   210 215 220
   Thr Ile Asp Asp Met Thr Lys Leu Arg Pro Val Phe Val Lys Glu Asn
   225 230 235 240
   Gly Thr Val Thr Ala Gly Asn Ala Ser Gly Leu Asn Asp Ala Ala Ala
   245 250 255
   Ala Val Val Met Met Glu Arg Ala Glu Ala Glu Arg Arg Gly Leu Lys
   260 265 270
   Pro Leu Ala Arg Leu Val Ser Tyr Gly His Ala Gly Val Asp Pro Lys
   275 280 285
   Ala Met Gly Ile Gly Pro Val Pro Ala Thr Lys Ile Ala Leu Glu Arg
   290 295 300
   Ala Gly Leu Gln Val Ser Asp Leu Asp Val Ile Glu Ala Asn Glu Ala
   305 310 315 320
   Phe Ala Ala Gln Ala Cys Ala Val Thr Lys Ala Leu Gly Leu Asp Pro
   325 330 335
   Ala Lys Val Asn Pro Asn Gly Ser Gly Ile Ser Leu Gly His Pro Ile
   340 345 350
   Gly Ala Thr Gly Ala Leu Ile Thr Val Lys Ala Leu His Glu Leu Asn
   355 360 365
   Arg Val Gln Gly Arg Tyr Ala Leu Val Thr Met Cys Ile Gly Gly Gly
   370 375 380
   Gln Gly Ile Ala Ala Ile Phe Glu Arg Ile
   385 390
<210> 54
   <211> 43
   <212> DNA
   <213> Artificial Sequence
<220>
   <223> Description of Artificial Sequence: oligonucleotide primer- A1 II up I
<400> 54
   ggaattcagg aggttttatg acgcgtgaag tggtagtggt aag 43
<210> 55
   <211> 29
   <212> DNA
   <213> Artificial Sequence
<220>
   <223> Description of Artificial Sequence: oligonucleotide primer- A1-II up II
<400> 55
   cgggatccga tacgctcgaa gatggcggc 29
<210> 56
   <211> 31
   <212> DNA
   <213> Artificial Sequence
<220>
   <223> Description of Artificial Sequence: oligonucleotide primer- A1-II dw I
<400> 56
   cgggatccac gcgtgaagtg gtagtggtaa g 31
<210> 57
   <211> 37
   <212> DNA
   <213> Artificial Sequence
<220>
   <223> Description of Artificial Sequence: oligonucleotide primer- A1-II dw II
<400> 57
   gctctagaag ctttcagata cgctcgaaga tggcggc 37
<210> 58
   <211> 1929
   <212> DNA
   <213> Ralstonia eutropha
<220>
   <221> gene
   <222> (1)..(1929)
   <223> bktB-linker-phbB fusion gene
<400> 58
   atgacgcgtg aagtggtagt ggtaagcggt gtccgtaccg cgatcgggac ctttggcggc 60
   agcctgaagg atgtggcacc ggcggagctg ggcgcactgg tggtgcgcga ggcgctggcg 120
   cgcgcgcagg tgtcgggcga cgatgtcggc cacgtggtat tcggcaacgt gatccagacc 180
   gagccgcgcg acatgtatct gggccgcgtc gcggccgtca acggcggggt gacgatcaac 240
   gcccccgcgc tgaccgtgaa ccgcctgtgc ggctcgggcc tgcaggccat tgtcagcgcc 300
   gcgcagacca tcctgctggg cgataccgac gtcgccatcg gcggcggcgc ggaaagcatg 360
   agccgcgcac cgtacctggc gccggcagcg cgctggggcg cacgcatggg cgacgccggc 420
   ctggtcgaca tgatgctggg tgcgctgcac gatcccttcc atcgcatcca catgggcgtg 480
   accgccgaga atgtcgccaa ggaatacgac atctcgcgcg cgcagcagga cgaggccgcg 540
   ctggaatcgc accgccgcgc ttcggcagcg atcaaggccg gctacttcaa ggaccagatc 600
   gtcccggtgg tgagcaaggg ccgcaagggc gacgtgacct tcgacaccga cgagcacgtg 660
   cgccatgacg ccaccatcga cgacatgacc aagctcaggc cggtcttcgt caaggaaaac 720
   ggcacggtca cggccggcaa tgcctcgggc ctgaacgacg ccgccgccgc ggtggtgatg 780
   atggagcgcg ccgaagccga gcgccgcggc ctgaagccgc tggcccgcct ggtgtcgtac 840
   ggccatgccg gcgtggaccc gaaggccatg ggcatcggcc cggtgccggc gacgaagatc 900
   gcgctggagc gcgccggcct gcaggtgtcg gacctggacg tgatcgaagc caacgaagcc 960
   tttgccgcac aggcgtgcgc cgtgaccaag gcgctcggtc tggacccggc caaggttaac 1020
   ccgaacggct cgggcatctc gctgggccac ccgatcggcg ccaccggtgc cctgatcacg 1080
   gtgaaggcgc tgcatgagct gaaccgcgtg cagggccgct acgcgctggt gacgatgtgc 1140
   atcggcggcg ggcagggcat tgccgccatc ttcgagcgta tcggatccat gactcagcgc 1200
   attgcgtatg tgaccggcgg catgggtggt atcggaaccg ccatttgcca gcggctggcc 1260
   aaggatggct ttcgtgtggt ggccggttgc ggccccaact cgccgcgccg cgaaaagtgg 1320
   ctggagcagc agaaggccct gggcttcgat ttcattgcct cggaaggcaa tgtggctgac 1380
   tgggactcga ccaagaccgc attcgacaag gtcaagtccg aggtcggcga ggttgatgtg 1440
   ctgatcaaca acgccggtat cacccgcgac gtggtgttcc gcaagatgac ccgcgccgac 1500
   tgggatgcgg tgatcgacac caacctgacc tcgctgttca acgtcaccaa gcaggtgatc 1560
   gacggcatgg ccgaccgtgg ctggggccgc atcgtcaaca tctcgtcggt gaacgggcag 1620
   aagggccagt tcggccagac caactactcc accgccaagg ccggcctgca tggcttcacc 1680
   atggcactgg cgcaggaagt ggcgaccaag ggcgtgaccg tcaacacggt ctctccgggc 1740
   tatatcgcca ccgacatggt caaggcgatc cgccaggacg tgctcgacaa gatcgtcgcg 1800
   acgatcccgg tcaagcgcct gggcctgccg gaagagatcg cctcgatctg cgcctggttg 1860
   tcgtcggagg agtccggttt ctcgaccggc gccgacttct cgctcaacgg cggcctgcat 1920
   atgggctga                                                         1929
<210> 59
   <211> 642
   <212> PRT
   <213> Artificial Sequence
<220>
   <223> Description of Artificial Sequence: Thiolase II Reductase Fusion Protein
<400> 59
   Met Thr Arg Glu Val Val Val Val Ser Gly Val Arg Thr Ala Ile Gly
   1               5                   10                  15
   Thr Phe Gly Gly Ser Leu Lys Asp Val Ala Pro Ala Glu Leu Gly Ala
               20                  25                  30
   Leu Val Val Arg Glu Ala Leu Ala Arg Ala Gln Val Ser Gly Asp Asp
           35                  40                  45
   Val Gly His Val Val Phe Gly Asn Val Ile Gln Thr Glu Pro Arg Asp
       50                  55                  60
   Met Tyr Leu Gly Arg Val Ala Ala Val Asn Gly Gly Val Thr Ile Asn
   65                  70                  75                  80
   Ala Pro Ala Leu Thr Val Asn Arg Leu Cys Gly Ser Gly Leu Gln Ala
                   85                  90                  95
   Ile Val Ser Ala Ala Gln Thr Ile Leu Leu Gly Asp Thr Asp Val Ala
               100                 105                 110
   Ile Gly Gly Gly Ala Glu Ser Met Ser Arg Ala Pro Tyr Leu Ala Pro
   115 120 125
   Ala Ala Arg Trp Gly Ala Arg Met Gly Asp Ala Gly Leu Val Asp Met
   130 135 140
   Met Leu Gly Ala Leu His Asp Pro Phe His Arg Ile His Met Gly Val
   145 150 155 160
   Thr Ala Glu Asn Val Ala Lys Glu Tyr Asp Ile Ser Arg Ala Gln Gln
   165 170 175
   Asp Glu Ala Ala Leu Glu Ser His Arg Arg Ala Ser Ala Ala Ile Lys
   180 185 190
   Ala Gly Tyr Phe Lys Asp Gln Ile Val Pro Val Val Ser Lys Gly Arg
   195 200 205
   Lys Gly Asp Val Thr Phe Asp Thr Asp Glu His Val Arg His Asp Ala
   210 215 220
   Thr Ile Asp Asp Met Thr Lys Leu Arg Pro Val Phe Val Lys Glu Asn
   225 230 235 240
   Gly Thr Val Thr Ala Gly Asn Ala Ser Gly Leu Asn Asp Ala Ala Ala
   245 250 255
   Ala Val Val Met Met Glu Arg Ala Glu Ala Glu Arg Arg Gly Leu Lys
   260 265 270
   Pro Leu Ala Arg Leu Val Ser Tyr Gly His Ala Gly Val Asp Pro Lys
   275 280 285
   Ala Met Gly Ile Gly Pro Val Pro Ala Thr Lys Ile Ala Leu Glu Arg
   290 295 300
   Ala Gly Leu Gln Val Ser Asp Leu Asp Val Ile Glu Ala Asn Glu Ala
   305 310 315 320
   Phe Ala Ala Gln Ala Cys Ala Val Thr Lys Ala Leu Gly Leu Asp Pro
   325 330 335
   Ala Lys Val Asn Pro Asn Gly Ser Gly Ile Ser Leu Gly His Pro Ile
   340 345 350
   Gly Ala Thr Gly Ala Leu Ile Thr Val Lys Ala Leu His Glu Leu Asn
   355 360 365
   Arg Val Gln Gly Arg Tyr Ala Leu Val Thr Met Cys Ile Gly Gly Gly
   370 375 380
   Gln Gly Ile Ala Ala Ile Phe Glu Arg Ile Gly Ser Met Thr Gln Arg
   385 390 395 400
   Ile Ala Tyr Val Thr Gly Gly Met Gly Gly Ile Gly Thr Ala Ile Cys
   405 410 415
   Gln Arg Leu Ala Lys Asp Gly Phe Arg Val Val Ala Gly Cys Gly Pro
   420 425 430
   Asn Ser Pro Arg Arg Glu Lys Trp Leu Glu Gln Gln Lys Ala Leu Gly
   435 440 445
   Phe Asp Phe Ile Ala Ser Glu Gly Asn Val Ala Asp Trp Asp Ser Thr
   450 455 460
   Lys Thr Ala Phe Asp Lys Val Lys Ser Glu Val Gly Glu Val Asp Val
   465 470 475 480
   Leu Ile Asn Asn Ala Gly Ile Thr Arg Asp Val Val Phe Arg Lys Met
   485 490 495
   Thr Arg Ala Asp Trp Asp Ala Val Ile Asp Thr Asn Leu Thr Ser Leu
   500 505 510
   Phe Asn Val Thr Lys Gln Val Ile Asp Gly Met Ala Asp Arg Gly Trp
   515 520 525
   Gly Arg Ile Val Asn Ile Ser Ser Val Asn Gly Gln Lys Gly Gln Phe
   530 535 540
   Gly Gln Thr Asn Tyr Ser Thr Ala Lys Ala Gly Leu His Gly Phe Thr
   545 550 555 560
   Met Ala Leu Ala Gln Glu Val Ala Thr Lys Gly Val Thr Val Asn Thr
   565 570 575
   Val Ser Pro Gly Tyr Ile Ala Thr Asp Met Val Lys Ala Ile Arg Gln
   580 585 590
   Asp Val Leu Asp Lys Ile Val Ala Thr Ile Pro Val Lys Arg Leu Gly
   595 600 605
   Leu Pro Glu Glu Ile Ala Ser Ile Cys Ala Trp Leu Ser Ser Glu Glu
   610 615 620
   Ser Gly Phe Ser Thr Gly Ala Asp Phe Ser Leu Asn Gly Gly Leu His
   625 630 635 640
   Met Gly
<210> 60
   <211> 1929
   <212> DNA
   <213> Ralstonia eutropha
<220>
   <221> gene
   <222> (1)..(1929)
   <223> phbB-linker-bktB fusion gene
<400> 60
   atgactcagc gcattgcgta tgtgaccggc ggcatgggtg gtatcggaac cgccatttgc 60
   cagcggctgg ccaaggatgg ctttcgtgtg gtggccggtt gcggccccaa ctcgccgcgc 120
   cgcgaaaagt ggctggagca gcagaaggcc ctgggcttcg atttcattgc ctcggaaggc 180
   aatgtggctg actgggactc gaccaagacc gcattcgaca aggtcaagtc cgaggtcggc 240
   gaggttgatg tgctgatcaa caacgccggt atcacccgcg acgtggtgtt ccgcaagatg 300
   acccgcgccg actgggatgc ggtgatcgac accaacctga cctcgctgtt caacgtcacc 360
   aagcaggtga tcgacggcat ggccgaccgt ggctggggcc gcatcgtcaa catctcgtcg 420
   gtgaacgggc agaagggcca gttcggccag accaactact ccaccgccaa ggccggcctg 480
   catggcttca ccatggcact ggcgcaggaa gtggcgacca agggcgtgac cgtcaacacg 540
   gtctctccgg gctatatcgc caccgacatg gtcaaggcga tccgccagga cgtgctcgac 600
   aagatcgtcg cgacgatccc ggtcaagcgc ctgggcctgc cggaagagat cgcctcgatc 660
   tgcgcctggt tgtcgtcgga ggagtccggt ttctcgaccg gcgccgactt ctcgctcaac 720
   ggcggcctgc atatgggcgg atccatgacg cgtgaagtgg tagtggtaag cggtgtccgt 780
   accgcgatcg ggacctttgg cggcagcctg aaggatgtgg caccggcgga gctgggcgca 840
   ctggtggtgc gcgaggcgct ggcgcgcgcg caggtgtcgg gcgacgatgt cggccacgtg 900
   gtattcggca acgtgatcca gaccgagccg cgcgacatgt atctgggccg cgtcgcggcc 960
   gtcaacggcg gggtgacgat caacgccccc gcgctgaccg tgaaccgcct gtgcggctcg 1020
   ggcctgcagg ccattgtcag cgccgcgcag accatcctgc tgggcgatac cgacgtcgcc 1080
   atcggcggcg gcgcggaaag catgagccgc gcaccgtacc tggcgccggc agcgcgctgg 1140
   ggcgcacgca tgggcgacgc cggcctggtc gacatgatgc tgggtgcgct gcacgatccc 1200
   ttccatcgca tccacatggg cgtgaccgcc gagaatgtcg ccaaggaata cgacatctcg 1260
   cgcgcgcagc aggacgaggc cgcgctggaa tcgcaccgcc gcgcttcggc agcgatcaag 1320
   gccggctact tcaaggacca gatcgtcccg gtggtgagca agggccgcaa gggcgacgtg 1380
   accttcgaca ccgacgagca cgtgcgccat gacgccacca tcgacgacat gaccaagctc 1440
   aggccggtct tcgtcaagga aaacggcacg gtcacggccg gcaatgcctc gggcctgaac 1500
   gacgccgccg ccgcggtggt gatgatggag cgcgccgaag ccgagcgccg cggcctgaag 1560
   ccgctggccc gcctggtgtc gtacggccat gccggcgtgg acccgaaggc catgggcatc 1620
   ggcccggtgc cggcgacgaa gatcgcgctg gagcgcgccg gcctgcaggt gtcggacctg 1680
   gacgtgatcg aagccaacga agcctttgcc gcacaggcgt gcgccgtgac caaggcgctc 1740
   ggtctggacc cggccaaggt taacccgaac ggctcgggca tctcgctggg ccacccgatc 1800
   ggcgccaccg gtgccctgat cacggtgaag gcgctgcatg agctgaaccg cgtgcagggc 1860
   cgctacgcgc tggtgacgat gtgcatcggc ggcgggcagg gcattgccgc catcttcgag 1920
   cgtatctga 1929
<210> 61
   <211> 642
   <212> PRT
   <213> Artificial Sequence
<220>
   <223> Description of Artificial Sequence: Reductase Thiolase II Fusion Protein
<220>
   <221> PEPTIDE
   <222> (1)..(642)
<400> 61
   Met Thr Gln Arg Ile Ala Tyr Val Thr Gly Gly Met Gly Gly Ile Gly
   1 5 10 15
   Thr Ala Ile Cys Gln Arg Leu Ala Lys Asp Gly Phe Arg Val Val Ala
   20 25 30
   Gly Cys Gly Pro Asn Ser Pro Arg Arg Glu Lys Trp Leu Glu Gln Gln
   35 40 45
   Lys Ala Leu Gly Phe Asp Phe Ile Ala Ser Glu Gly Asn Val Ala Asp
   50 55 60
   Trp Asp Ser Thr Lys Thr Ala Phe Asp Lys Val Lys Ser Glu Val Gly
   65 70 75 80
   Glu Val Asp Val Leu Ile Asn Asn Ala Gly Ile Thr Arg Asp Val Val
   85 90 95
   Phe Arg Lys Met Thr Arg Ala Asp Trp Asp Ala Val Ile Asp Thr Asn
   100 105 110
   Leu Thr Ser Leu Phe Asn Val Thr Lys Gln Val Ile Asp Gly Met Ala
   115 120 125
   Asp Arg Gly Trp Gly Arg Ile Val Asn Ile Ser Ser Val Asn Gly Gln
   130 135 140
   Lys Gly Gln Phe Gly Gln Thr Asn Tyr Ser Thr Ala Lys Ala Gly Leu
   145 150 155 160
   His Gly Phe Thr Met Ala Leu Ala Gln Glu Val Ala Thr Lys Gly Val
   165 170 175
   Thr Val Asn Thr Val Ser Pro Gly Tyr Ile Ala Thr Asp Met Val Lys
   180 185 190
   Ala Ile Arg Gln Asp Val Leu Asp Lys Ile Val Ala Thr Ile Pro Val
   195 200 205
   Lys Arg Leu Gly Leu Pro Glu Glu Ile Ala Ser Ile Cys Ala Trp Leu
   210 215 220
   Ser Ser Glu Glu Ser Gly Phe Ser Thr Gly Ala Asp Phe Ser Leu Asn
   225 230 235 240
   Gly Gly Leu His Met Gly Gly Ser Met Thr Arg Glu Val Val Val Val
   245 250 255
   Ser Gly Val Arg Thr Ala Ile Gly Thr Phe Gly Gly Ser Leu Lys Asp
   260 265 270
   Val Ala Pro Ala Glu Leu Gly Ala Leu Val Val Arg Glu Ala Leu Ala
   275 280 285
   Arg Ala Gln Val Ser Gly Asp Asp Val Gly His Val Val Phe Gly Asn
   290 295 300
   Val Ile Gln Thr Glu Pro Arg Asp Met Tyr Leu Gly Arg Val Ala Ala
   305 310 315 320
   Val Asn Gly Gly Val Thr Ile Asn Ala Pro Ala Leu Thr Val Asn Arg
   325 330 335
   Leu Cys Gly Ser Gly Leu Gln Ala Ile Val Ser Ala Ala Gln Thr Ile
   340 345 350
   Leu Leu Gly Asp Thr Asp Val Ala Ile Gly Gly Gly Ala Glu Ser Met
   355 360 365
   Ser Arg Ala Pro Tyr Leu Ala Pro Ala Ala Arg Trp Gly Ala Arg Met
   370 375 380
   Gly Asp Ala Gly Leu Val Asp Met Met Leu Gly Ala Leu His Asp Pro
   385 390 395 400
   Phe His Arg Ile His Met Gly Val Thr Ala Glu Asn Val Ala Lys Glu
   405 410 415
   Tyr Asp Ile Ser Arg Ala Gln Gln Asp Glu Ala Ala Leu Glu Ser His
   420 425 430
   Arg Arg Ala Ser Ala Ala Ile Lys Ala Gly Tyr Phe Lys Asp Gln Ile
   435 440 445
   Val Pro Val Val Ser Lys Gly Arg Lys Gly Asp Val Thr Phe Asp Thr
   450 455 460
   Asp Glu His Val Arg His Asp Ala Thr Ile Asp Asp Met Thr Lys Leu
   465 470 475 480
   Arg Pro Val Phe Val Lys Glu Asn Gly Thr Val Thr Ala Gly Asn Ala
   485 490 495
   Ser Gly Leu Asn Asp Ala Ala Ala Ala Val Val Met Met Glu Arg Ala
   500 505 510
   Glu Ala Glu Arg Arg Gly Leu Lys Pro Leu Ala Arg Leu Val Ser Tyr
   515 520 525
   Gly His Ala Gly Val Asp Pro Lys Ala Met Gly Ile Gly Pro Val Pro
   530 535 540
   Ala Thr Lys Ile Ala Leu Glu Arg Ala Gly Leu Gln Val Ser Asp Leu
   545 550 555 560
   Asp Val Ile Glu Ala Asn Glu Ala Phe Ala Ala Gln Ala Cys Ala Val
   565 570 575
   Thr Lys Ala Leu Gly Leu Asp Pro Ala Lys Val Asn Pro Asn Gly Ser
   580 585 590
   Gly Ile Ser Leu Gly His Pro Ile Gly Ala Thr Gly Ala Leu Ile Thr
   595 600 605
   Val Lys Ala Leu His Glu Leu Asn Arg Val Gln Gly Arg Tyr Ala Leu
   610 615 620
   Val Thr Met Cys Ile Gly Gly Gly Gln Gly Ile Ala Ala Ile Phe Glu
   625 630 635 640
   Arg Ile

## Description

### Background Of The Invention

The present invention is generally in the field of genetically engineered bacterial and plant systems for production of polyhydroxyalkanoates by microorganisms and genetically engineered plants, wherein the enzymes essential for production of the polymers are expressed as fusion proteins having enhanced properties for polymer synthesis.

Numerous microorganisms have the ability to accumulate intracellular reserves of poly[(*R*)-3-hydroxyalkanoate] polymers or PHAs. PHAs are biodegradable and biocompatible thermoplastic materials with a broad range of industrial and biomedical applications (Williams and Peoples, 1996, CHEMTECH 26, 38-44). In recent years, the PHA biopolymers have emerged from what was originally considered to be a single homopolymer, poly-3-hydroxybutyrate (PHB), into a broad class of polyesters with different monomer compositions and a wide range of physical properties. Over 100 different monomers have been incorporated into the PHA polymers (Steinbüchel and Valentin, 1995, FEMS Microbiol. Lett. 128; 219-228). It has been useful to divide the PHAs into two groups according to the length of their side chains and their biosynthetic pathways. Those with short side chains, such as polyhydroxybutyrate (PHB), a homopolymer of *R*-3-hydroxybutyric acid units, are semi-crystalline thermoplastics, whereas PHAs with long side chains are more elastomeric.

Biosynthesis of the short side-chain PHAs such as PHB and PHBV proceeds through a sequence of three enzyme catalyzed reactions from the central metabolite acetyl-CoA. In the first step of this pathway, two acetyl-CoA molecules are condensed to acetoacetyl-CoA by a 3-ketoacyl-CoA thiolase. Acetoacetyl-CoA is subsequently reduced to the PHB precursor 3-hydroxybutyryl-CoA by an NADPH dependent reductase. 3-hydroxybutyryl-CoA is then polymerized to PHB which is sequestered by the bacteria as "intracellular inclusion bodies" or granules. The molecular weight of PHB is generally in the order of 10⁴-10⁷ Da. In some bacteria such as *Chromatium vinosum* the reductase enzyme is active primarily with NADH as co-factor. The synthesis of the PHBV co-polymer proceeds through the same pathway, with the difference being that acetyl-CoA and propionyl-CoA are converted to 3-ketovaleryl-CoA by β-ketothiolase. 3-ketovaleryl-CoA is then converted to 3-hydroxyvaleryl-CoA which is polymerized.

Long side chain PHAs are produced from intermediates of fatty acid β-oxidation or fatty acid biosynthesis pathways. In the case of β-oxidation, the L-isomer of β-hydroxyacyl-CoA is converted to the D-isomer by an epimerase activity present on the multi-enzyme complex encoded by the *fao*AB genes. Biosynthesis from acetyl-CoA through the fatty acid synthase route produces the L-isomer of β-hydroxyacyl-ACP. Conversion of the ACP to the CoA derivative is catalyzed by the product of the *pha*G gene (Kruger and Steinbuchel 1998, U.S. patent 5,750,848).

Enoyl-CoA hydratases have been implicated in PHA biosynthesis in microbes such as *Rhodospirillum rubrum* and *Aeromonas caviae.* The biosynthesis of PHB in *R. rubrum* is believed to proceed through an acetoacetyl-CoA reductase enzyme specific for the L-isomer of 3-hydroxybutyryl-CoA. Conversion of the L to the D form is then catalysed by the action of two enoyl-CoA hydratase activities. In the case of the PHB-co-HX, where X is a C6-C16 hydroxy acid, copolymers which are usually produced from cells grown on fatty acids, a combination of these routes can be responsible for the formation of the different monomeric units. Indeed, analysis of the DNA locus encoding the PHA synthase gene in *Aeromonas caviae,* which produces the copolymer PHB-co-3-hydroxyhexanoate, identified a gene encoding a D-specific enoyl-CoA hydratase responsible for the production of the D-β-hydroxybutyryl-CoA and D-β-hydroxyhexanoyl-CoA units (Fukui and Doi, 1997, J. Bacteriol. 179: 4821-4830; Fukui et. al., 1998, J. Bacteriol. 180: 667-673).

It is desirable for economic reasons to be able to produce these polymers in transgenic crop species. Methods for achieving this are known. See, for example, U.S. patent NO. 5 ,245,023 and U.S. patent NO. 5 ,250,430; U.S. patent NO. 5 ,502,273; U.S. patent NO. 5 ,534,432; U.S. patent NO. 5 ,602,321; U.S. patent NO. 5,610,041; U.S. patent NO. 5 ,650,555: U.S. patent NO.5 ,663,063; WO, 9100917, WO 9219747, WO 9302187, WO 9302194 and WO 9412014, Poirier et.al., 1992, Science 256; 520-523, Williams and Peoples, 1996, Chemtech 26, 38-44. In order to achieve this goal, it is necessary to transfer a gene, or genes in the case of a PHA synthase with more than one subunit, encoding a PHA synthase from a microorganism into plant cells and obtain the appropriate level of production of the PHA synthase enzyme. In addition it may be necessary to provide additional PHA biosynthetic genes, eg. a ketoacyl-CoA thiolase, an acetoacetyl-CoA reductase gene, a 4-hydroxybutyryl-CoA transferase gene or other genes encoding enzymes required to synthesize the substrates for the PHA synthase enzymes.

In many cases, it is particularly desirable to control the expression in different plant tissues or organelles. Methods for controlling expression are known to those skilled in the art (Gasser and Fraley, 1989, Science 244; 1293-1299; Gene Transfer to Plants,1995, Potrykus, I. and Spangenberg, G. eds. Springer -Verlag Berlin Heidelberg New York. and "Transgenic Plants: A Production System for Industrial and Pharmaceutical Proteins", 1996, Owen, M.R.L. and Pen, J. Eds. John Wiley & Sons Ltd. England). U.S. PATENT NO. 5 ,610,041 describes the route of plastid expression by the previously known technology of adding a leader peptide to direct the protein expressed from the nuclear gene to the plastid. More recent technology enables the direct insertion of foreign genes directly into the plastid chromosome by recombination (Svab *et al.,* 1990, Proc. Natl. Acad. Sci. USA. 87: 8526-8530; McBride et al., 1994, Proc. Natl. Acad. Sci. USA. 91: 7301-7305). The prokaryotic nature of the plastid RNA and protein synthesis machinery also allows for the expression of microbial genes such as for example the *phbC, phbA* and *phbB* genes of *R. eutropha.*

Genetic engineering of bacteria and plants to make products such as polymers which require the coordinated expression and action of multiple enzymes, sequentially on different substrates, may result in low yields, or poor efficiencies, or variations or deviation in the final product.

It is therefore an object of the present invention to provide methods and materials for enhancing production of products of multiple enzymes, such as polymers, and particularly polyhydroxyalkanoates, in bacteria or plants.

### Summary of the Invention

In order to optimize the flux or flow of carbon intermediates from normal cellular metabolism into PHAs it is desirable to optimize the expression of the enzymes of the PHA biosynthetic pathway. Gene fusions are genetic constructs where two open reading frames have been fused into one. The transcriptional and translational sequences upstream of the first open reading frame direct the synthesis of a single protein with the primary structure that comprises both original open reading frames. Consequently, gene fusions encode hybrid proteins and in some cases bifunctional hybrid enzymes. Individual genes are isolated, for example, by PCR, such that the resulting DNA fragments contain the complete coding region or parts of the coding region of interest. The DNA fragment that encodes the amino-terminal domain of the hybrid protein may contain a translation initiation site and a transcriptional control sequence. The stop codon in the gene encoding the amino-terminal domain needs to be removed from this DNA fragment. The stop codon in the gene encoding the carboxy-terminal domain needs to be retained in the DNA fragment. DNA sequences that are recognized by restriction enzymes may be introduced into the new genes for DNA cloning purposes. Linkers may be added to spatially separate the two domains of the hybrid protein.

In the case of enzymes which catalyse successive reactions in a pathway, the fusion of two genes results in bringing two enzymatic activities into close proximity to each other. When the product of the first reaction is a substrate for the second one, this new configuration of active sites may result in a faster transfer of the product of the first reaction to the second active site with a potential for increasing the flux through the pathway. The configuration of the two catalytic domains in the hybrid in relation to one another, may be altered by providing a linker sequence between them. This linker may be composed of any of the twenty natural amino acids and can be of variable length. The variation in length and composition are important parameters for changing the relative configuration of the individual domains of the hybrid and its enzyme activities.

This technology allows for the direct incorporation of a series of genes encoding a multi-enzyme pathway into a bacteria or plant or plant organelle, for example, the plastid genome. In some cases it may be useful to re-engineer the 5' -untranslated regions of plastid genes which are important for mRNA stability and translation (Hauser et al., 1996. J. Biol. Chem. 271: 1486-1497), remove secondary structure elements, or add elements from highly expressed plastid genes to maximize expression of transgenes encoded by an operon.

Examples demonstrate the expression of active polypeptides encoding multiple enzyme activies. These are homotetrameric enzymes which require the use of cofactors and which interact to synthesize polymer, which have not previously been demonstrated to be expressable as fusion proteins.

### Brief Description of the Drawings

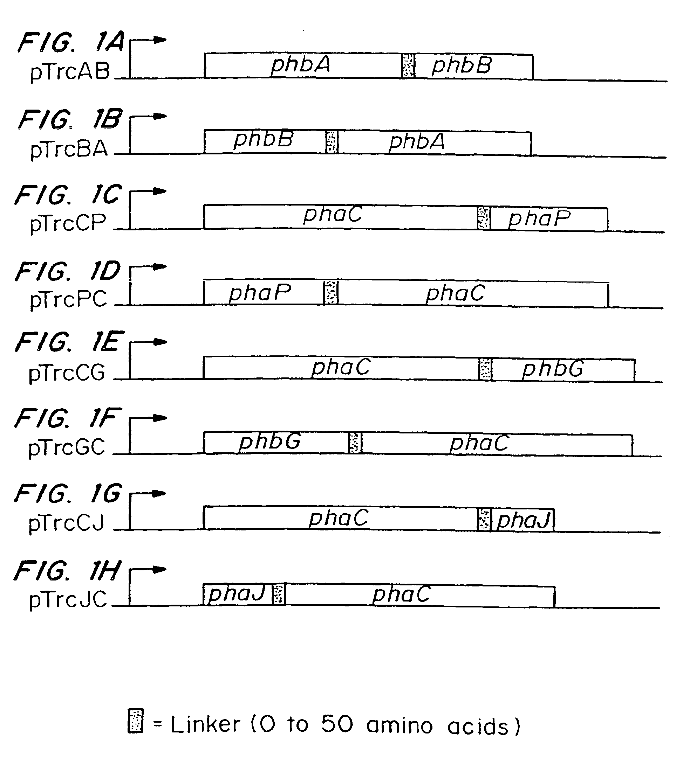

Figures 1A-1H are schematics of gene fusions encoding multiple-enzyme proteins: pTrcAB including beta-ketothiolase (phbA) and acyl-CoA reductase (phbB) (1A); pTrcBA including phbB and phbA (1B); pTrcCP including PHA synthase (phaC) and phasin (phaP) (1C); pTrcPC including phaP and phaC (1D); pTrcCG including phaC and beta-hydroxyacyl-ACP::coenzyme-A transferase (phbG) (1E); pTrcGC including phbG and phaC (IF); pTrcCJ including phaC and enoyl-CoA hydratases (phaJ) (1G); and pTrcJC including phaJ and phaC (1H).

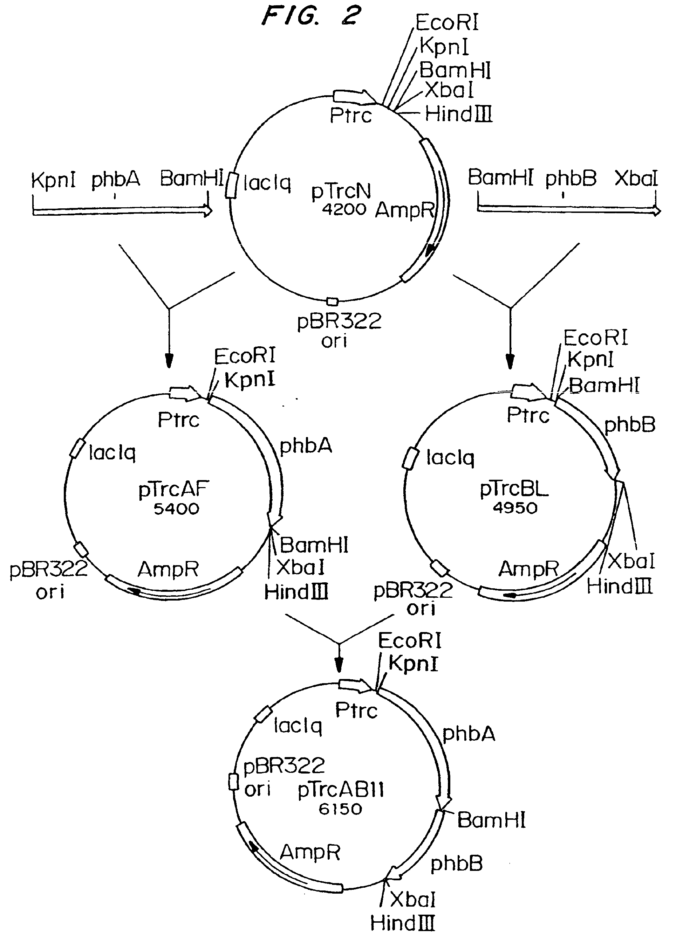

Figure 2 is a schematic of the construction of pTrcAB11, including phbA and phbB, on a single polypeptide with both thiolase and reductase activity.

### Detailed Description of the Invention

### I. Gene Fusions

In order to optimize the flux or flow of carbon intermediates from normal cellular metabolism into PHAs it is desirable to optimize the expression of the enzymes of the PHA biosynthetic pathway. Gene fusions are genetic constructs where two open reading frames have been fused into one. The transcriptional and translational sequences upstream of the first open reading frame direct the synthesis of a single protein with the primary structure that comprises both original open reading frames. Consequently, gene fusions encode hybrid proteins and in some cases bifunctional hybrid enzymes. Hybrid proteins have been developed for applications such as protein purification (Bülow, L., Eur. J. Biochem. (1987) 163: 443-448; Bülow, L., Biochem. Soc. Symp. (1990) 57: 123-133); Bülow, L., Tibtech.(1991) 9: 226-231), biochemical analyses (Ljungcrantz et al. FEBS Lett. (1990) 275: 91-94; Ljungcrantz et al., Biochemistry (1989) 28: 8786-8792; Bülow, L., Biochem. Soc. Symp. (1990) 57: 123-133); Bülow, L., Tibtech.(1991) 9: 226-231) and metabolic engineering (U.S. Patent 5,420, 027; Carlsson, Biotech. Lett. (1992) 14: 439-444; Bülow, L., Biochem. Soc. Symp. (1990) 57: 123-133); Bulow, L., Tibtech.(1991) 9: 226-231; Fisher, Proc. Natl. Acad. Sci. U.S.A. (1992) 89: 10817-10821).

Individual genes are isolated, for example, by PCR, such that the resulting DNA fragments contain the complete coding region or parts of the coding region of interest. The DNA fragment that encodes the amino-terminal domain of the hybrid protein may contain a translation initiation site and a transcriptional control sequence. The stop codon in the gene encoding the amino-terminal domain needs to be removed from this DNA fragment. The stop codon in the gene encoding the carboxy-terminal domain needs to be retained in the DNA fragment. DNA sequences that are recognized by restriction enzymes may be introduced into the new genes for DNA cloning purposes. Linkers may be added to spatially separate the two domains of the hybrid protein.

In the case of enzymes which catalyse successive reactions in a pathway, the fusion of two genes results in bringing two enzymatic activities into close proximity to each other. When the product of the first reaction is a substrate for the second one, this new configuration of active sites may result in a faster transfer of the product of the first reaction to the second active site with a potential for increasing the flux through the pathway. The configuration of the two catalytic domains in the hybrid in relation to one another, may be altered by providing a linker sequence between them. This linker may be composed of any of the twenty natural amino acids and can be of variable length. The variation in length and composition are important parameters for changing the relative configuration of the individual domains of the hybrid and its enzyme activities.

Methods exist for improving the utility of PHA biosynthetic fusion enzymes using molecular evolution or "gene-shuffling" techniques (Stemmer, M.P.C. 1994, Nature, 370: 389-391; Stemmer, M.P.C. 1994, Proc. Natl. Acad. Sci., 1994, 91: 10747-10751). Requirements to make this approach work include the mutagenesis techniques, which are usually PCR-based, and a screening technique to identify those mutant enzymes with the desired improved properties.

### A. Genes

Suitable genes include PHB and PHA synthases, β-ketothiolases, acyl-CoA reductases, phasins, enoyl-CoA hydratases and β-hydroxyacyl-ACP::coenzyme-A transferases. Examples of fusions that can be constructed are illustrated in Figures 1A-1H.

β-ketothiolase encoding genes have been isolated from *Alcaligenes latus* (MBX unpublished; Choi, et al. Appl. Environ. Micrbiol. 64 (12), 4897-4903 (1998)), *Ralstonia eutropha* [Peoples, O.P. and Sinskey, A.J., J. Biol. Chem. 264: 15298-15303 (1989); Slater et. al., 1998, J. Bacteriol. 180: 1979-1987], *Acinetobacter* sp. [Schembri, et al. J. Bacteriol., *Chromatium vinosum* [Liebergesell, M. and Steinbuchel, A. Eur. J. Biochem. 209 (1), 135-150 (1992)], *Pseudomonas acidophila* (Umeda, et al. Appl. Biochem. Biotech. 70-72: 341-352 (1998)], *Pseudomonas denitrificans* [Yabutani, et al. FEMS Microbiol. Lett. 133 (1-2), 85-90 (1995)], *Rhizobium meliloti* [Tombolini, et al. Microbiology 141, 2553-2559 (1995)], *Thiocystis violacea* [Liebergesell, et al. Appl. Microbiol. Biotechnol. 38 (4), 493-501 (1993)], and *Zoogloea ramigera* [Peoples, et al. J. Biol. Chem. 262 (1), 97-102 (1987)].

Reductase encoding genes have been isolated from *Alcaligenes latus* (Choi, et al. Appl. Environ. Micrbiol. 64 (12), 4897-4903 (1998)], *R*. *eutropha* [Peoples, O.P. and Sinskey, A.J., J. Biol. Chem. 264 (26), 15298-15303 (1989); *Acinetobacter* sp. (Schembri, et al. J. Bacteriol), *C*. *vinosum* [Liebergesell, M. and Steinbuchel, A. Eur. J. Biochem. 209 (1), 135-150 (1992)], *Pseudomonas acidophila* (Umeda, et al. Appl. Biochem. Biotech. 70-72: 341-352 (1998)], *P. denitrificans* [Yabutani, et al. FEMS Microbiol. Lett. 133 (1-2), 85-90 (1995)], *R. metiloti* [Tombolini, et al. Microbiology 141 (Pt 10), 2553-2559 (1995)], and Z. *ramigera* [Peoples, O.P. and Sinskey, A.J., 1989, Molecular Microbiology, 3: 349-357).

PHA synthase encoding genes have been isolated from *Aeromonas caviae* [Fukui, T. and Doi, Y. J. Bacteriol. 179 (15), 4821-4830 (1997)], *Alcaligenes latus* (Choi, et al. Appl. Environ. Microbiol. 64 (12), 4897-4903 (1998)], *R. eutropha* [Peoples, O.P. and Sinskey, A.J. J. Biol. Chem. 264 (26), 15298-15303 (1989); Lee, et al. *Acinetobacter* [Schembri, et al. J. Bacteriol.], *C*. *vinosum* [Liebergesell, M. and Steinbuchel, A. Eur. J. Biochem. 209 (1), 135-150 (1992)], *Methylobacterium extorquens* [Valentin, and Steinbuchel, Appl. Microbiol. Biotechnol. 39 (3), 309-317 (1993)], *Nocardia corallina* (GenBank Acc. No. AF019964), *Nocardia salmonicolor, Pseudomonas acidophila* (Umeda, et al. T. Appl. Biochem. Biotech. 70-72: 341-352 (1998)], *P. denitrificans* [Ueda, et al. J. Bacteriol. 178 (3), 774-779 (1996)], *Pseudomonas aeruginosa* [Timm, and Steinbuchel, Eur. J. Biochem. 209 (1), 15-30 (1992)], *Pseudomonas oleovorans* [Huisman, et al. J. Biol. Chem. 266 (4), 2191-2198 (1991)], *Rhizobium etli* [Cevallos, et al. J. Bacteriol. 178 (6), 1646-1654 (1996)], *R. meliloti* [Tombolini, et al. Microbiology 141 (Pt 10), 2553-2559 (1995)], *Rhodococcus ruber* [Pieper, U. and Steinbuechel, A. FEMS Microbiol.Lett. 96 (1), 73-80 (1992)], *Rhodospirrilum rubrum* [Hustede, et al. FEMS Microbiol. Lett. 93, 285-290 (1992)], *Rhodobacter sphaeroides* [Steinbüchel, et al. FEMS Microbiol. Rev. 9 (2-4), 217-230 (1992); Hustede, et al. Biotechnol. Lett. 15, 709-714 (1993)], *Synechocystis* sp. [Kaneko, T., DNA Res. 3 (3), 109-136 (1996)], *T. violaceae* [Liebergesell, et al. Appl. Microbiol. Biotechnol. 38 (4), 493-501 (1993)], and *Z. ramigera* (GenBank Acc. No. U66242).

Other genes that have not been implicated in PHA formation but which share significant homology with the *phb* genes and/or the corresponding gene products may be used as well. Genes encoding thiolase and reductase like enzymes have been identified in a broad range of non-PHB producing bacteria. *E. coli* (U29581, D90851, D90777), *Haemophilus influenzae* (U32761), *Pseudomonas fragi* (D10390), *Pseudomonas aeruginosa* (U88653), *Clostridium acetobutylicum* (U08465), *Mycobacterium leprae* (U00014), *Mycobacterium tuberculosis* (Z73902), *Helicobacter pylori* (AE000582), *Thermoanaerobacterium thermosaccharolyticum* (Z92974), *Archaeoglobus fulgidus* (AE001021), *Fusobacterium nucleatum* (U37723), *Acinetobacter calcoacelicus* (L05770), *Bacillus subtilis* (D84432, Z99120, U29084) and *Synechocystis* sp. (D90910) all encode one or more thiolases from their chromosome. Eukaryotic organisms such as *Saccharomyces cerevisiae* (L20428), *Schizosaccharomyces pombe* (D89184), *Candida tropicalis* (D13470), *Caenorhabditis elegans* (U41105), human (S70154), rat (D13921), mouse (M35797), radish (X78116), pumpkin (D70895) and cucumber (X67696) also express proteins with significant homology to the 3-ketothiolase from *R*. *eutropha.*

Genes with significant homology to the *phbB* gene encoding acetoacetyl CoA reductase have been isolated from several organisms: *Azospirillum brasiliense* (X64772, X52913) and *Rhizobium* sp. (U53327, Y00604), *E. coli* (D90745), *Vibrio harveyi* (U39441), *H. influenzae* (U32701), *B. subtilis* (U59433), *P. aeruginosa* (U91631), *Synechocystis* sp. (D90907), *H pylori* (AE000570), *Arabidopsis thaliana* (X64464), *Cuphea lanceolata* (X64566) and *Mycobacterium smegmatis* (U66800).

A number of proteins which bind to PHA granules have been identified and their genes cloned (Steinbuchel et. al., 1995, Can. J. Microbiol. (Supplement 1) 41:94-105). The current hypothesis is that these proteins play a role similar to the oleosin oil storage proteins (Huang, A.H.C. 1992, Annu. Rev. Plant Physiol. Plant Mol. Biol. 43: 177-200) in oilseeds and have been named phasins. For example, protein GA24 is a 24 kilodalton protein found in PHA producing cells of *Alcaligenes eutrophus* (Wieczorek et al., J. Bacteriol. 1995, 177, 2425-2435). The gene encoding GA24, *phaP,* has been isolated by complementation of PHA-leaky mutants of the bacterium. Wieczorek et al., in their studies of GA24, observed that the protein coated PHA granules in PHA producing cells of *A*. *eutrophus,* and that cells deficient in GA24 formed very large granules whereas wild-type cells possessed much smaller granules (Wieczorek et al., J. Bacteriol. 1995, 177, 2425-2435). Based on this observation, the authors proposed that GA24 is one of a number of such proteins termed phasins responsible for controlling PHA granule size. An immunological analysis of other PHA granules from a number of different bacteria indicated conservation of this protein (Wieczorek et. al., 1996, FEMS Microbiology letters 135: 23-30) and the authors concluded that homologs to GA24 are widespread and their genes can be readily isolated. A 13Kd phasin has been identified in *Acinetobacter sp.* (Schembri et. al., 1995, FEMS Micro. Lett. 133: 277-283).

### B. Transformation Vectors

DNA constructs include transformation vectors capable of introducing transgenes into plants. There are many plant transformation vector options available. See (Gene Transfer to Plants (1995), Potrykus, I. and Spangenberg, G. eds. Springer -Verlag Berlin Heidelberg New York; "Transgenic Plants: A Production System for Industrial and Pharmaceutical Proteins" (1996), Owen, M.R.L. and Pen, J. eds. John Wiley & Sons Ltd. England and Methods in Plant Molecular Biology-a laboratory course manual (1995), Maliga, P., Klessig, D.F., Cashmore, A. R., Gruissem, W. and Varner, J.E. eds. Cold Spring Laboratory Press, New York).

### C. Regulatory Sequences

In general, plant transformation vectors comprise one or more coding sequences of interest under the transcriptional control of 5' and 3' regulatory sequences, including a promoter, a transcription termination and/or polyadenylation signal and a selectable or screenable marker gene. The usual requirements for 5' regulatory sequences include a promoter, a transcription initiation site, and a mRNA processing signal. 3' regulatory sequences include a transcription termination and/or a polyadenylation signal. Additional RNA processing signals and ribozyme sequences can be engineered into the construct for the expression of two or more polypeptides from a single transcript (U.S. PATENT NO. 5 ,519,164). This approach has the advantage of locating multiple transgenes in a single locus which is advantageous in subsequent plant breeding efforts. An additional approach is to use a vector to specifically transform the plant plastid chromosome by homologous recombination (US 5,545,818), in which case it is possible to take advantage of the prokaryotic nature of the plastid genome and insert a number of transgenes as an operon.

A large number of plant promoters are known and result in either constitutive, or environmentally or developmentally regulated expression of the gene of interest. Plant promoters can be selected to control the expression of the transgene in different plant tissues or organelles, as described by (Gasser and Fraley, 1989, Science 244; 1293-1299). The 5' end of the transgene may be engineered to include sequences encoding plastid or other subcellular organelle targeting peptides linked in-frame with the transgene. Suitable constitutive plant promoters include the cauliflower mosaic virus 35S promoter (CaMV) and enhanced CaMV promoters (Odell et. al., 1985, Nature, 313: 810), actin promoter (McElroy et al., 1990, Plant Cell 2: 163-171), AdhI promoter (Fromm et. al., 1990, Bio/Technology 8: 833-839; Kyozuka et al., 1991, Mol. Gen. Genet. 228: 40-48), ubiquitin promoters, the Figwort mosaic virus promoter, mannopine synthase promoter, nopaline synthase promoter and octopine synthase promoter. Useful regulatable promoter systems include spinach nitrate-inducible promoter, heat shock promoters, small subunit of ribulose biphosphate carboxylase promoters and chemically inducible promoters (U.S. Patent NO. 5,364,780 and U.S. Patent NO. 5 ,364,780).

It may be preferable to express the transgenes only in the developing seeds. Promoters suitable for this purpose include the napin gene promoter (U.S. PATENT NO. 5 ,420,034; U.S. PATENT NO. 5 ,608,152), the acetyl-CoA carboxylase promoter (U.S. PATENT NO. 5 ,420,034; U.S. PATENT NO. 5 ,608,152), 2S albumin promoter, seed storage protein promoter, phaseolin promoter (Slightom et. al., 1983, Proc, Natl. Acad. Sci. USA 80: 1897-1901), oleosin promoter (plant et. al., 1994, Plant Mol. Biol. 25: 193-205; Rowley et. al., 1997, Biochim. Biophys. Acta.1345: 1-4; U.S. PATENT NO. 5 ,650,554; PCT WO 93/20216), zein promoter, glutelin promoter, starch synthase promoter, and starch branching enzyme promoter.

A number of useful plant vectors comprising many of the features described above have been described in the literature. Particularly useful among these are the "super-binary" vectors described by Ishida et. al., (1996, Nature biotechnology 14: 745-750) and the extensive range of vectors available from Cambia, Canberra, Australia (described by Roberts et. al., "A comprehensive set of modular vectors for advanced manipulations and efficient transformation of plants" presented at the Rockefeller Foundation Meeting of the International Program on Rice Biotechnology, 15-18 September 1997, Malacca, Malaysia).

### II. Methods for Transformation of Plants and Selection Thereof

It is preferable to express more than one gene product in the plant. A number of methods can be used to achieve this including: introducing the encoding DNAs in a single transformation event where all necessary DNAs are on a single vector; in a co-transformation event where all necessary DNAs are on separate vectors but introduced into plant cells simultaneously; introducing the encoding DNAs by independent transformation events successively into the plant cells i.e. transformation of transgenic plant cells expressing one or more of the encoding DNAs with additional DNA constructs; transformation of each of the required DNA constructs by separate transformation events, obtaining transgenic plants expressing the individual proteins and using traditional plant breeding methods to incorporate the entire pathway into a single plant.

The transformation of suitable agronomic plant hosts using these vectors can be accomplished by a range of methods and plant tissues. Suitable plants include: the *Brassica* family including *napus, rappa, sp. carinata* and *juncea,* maize, soybean, cottonseed, sunflower, palm, coconut, safflower, peanut, mustards including *Sinapis alba* and flax. Suitable tissues for transformation using these vectors include protoplasts, cells, callus tissue, leaf discs, pollen, meristems etc. Suitable transformation procedures include *Agrobacterium-*mediated transformation, biolistics, microinjection, electroporation, polyethylene glycol-mediated protoplast transformation, liposome-mediated transformation, silicon fiber-mediated transformation (U.S. PATENT NO. 5 ,464,765) etc. (Gene Transfer to Plants (1995), Potrykus, I. and Spangenberg, G. eds. Springer -Verlag Berlin Heidelberg New York; "Transgenic Plants: A Production System for Industrial and Pharmaceutical Proteins" (1996), Owen, M.R.L. and Pen, J. eds. John Wiley & Sons Ltd. England and Methods in Plant Molecular Biology-a laboratory course manual (1995), Maliga, P., Klessig, D.F., Cashmore, A. R., Gruissem, W. and Varner, J.E. eds. Cold Spring Laboratory Press, New York).

Transformation procedures have been established for these specific crops (Gene Transfer to Plants (1995), Potrykus, I. and Spangenberg, G. eds. Springer -Verlag Berlin Heidelberg New York; "Transgenic Plants: A Production System for Industrial and Pharmaceutical Proteins" (1996), Owen, M.R.L. and Pen, J. eds. John Wiley & Sons Ltd. England and Methods in Plant Molecular Biology-A laboratory course manual (1995), Maliga, P., Klessig, D.F., Cashmore, A. R., Gruissem, W. and Varner, J.E. eds. Cold Spring Laboratory Press, New York).

*Brassica napus* can be transformed as described for example in U.S. PATENT NO. 5 ,188,958 and U.S. PATENT NO. 5 ,463,174. Other *Brassica* such as *rappa, carinata* and *juncea* as well as *Sinapis alba* can be transformed as described by Moloney et. al., (1989, Plant Cell Reports 8: 238-242). Soybean can be transformed by a number of reported procedures. See (U.S. PATENT NO. 5 ,015,580; U.S. PATENT NO. 5 ,015,944; U.S. PATENT NO. 5 ,024,944; U.S. PATENT NO. 5 ,322,783; U.S. PATENT NO. 5 ,416,011; U.S. PATENT NO. 5 ,169,770). A number of transformation procedures have been reported for the production of transgenic maize plants including pollen transformation (U.S. PATENT NO. 5 ,629,183), silicon fiber-mediated transformation (U.S. PATENT NO. 5 ,464,765) electroporation of protoplasts (U.S. PATENT NO. 5 ,231,019; U.S. PATENT NO. 5 ,472,869; U.S. PATENT NO. 5 ,384,253) gene gun (U.S. PATENT NO. 5 ,538,877; U.S. PATENT NO. 5,538,880 and *Agrobacterium*-mediated transformation (EP 0 604 662 A1; WO 94/00977). The *Agrobacterium*-mediated procedure is particularly preferred as single integration events of the transgene constructs are more readily obtained using this procedure which greatly facilitates subsequent plant breeding. Cotton can be transformed by particle bombardment (U.S. PATENT NO. 5 ,004,863; U.S. PATENT NO. 5 ,159,135). Sunflower can be transformed using a combination of particle bombardment and *Agrobacteriuim* infection (EP 0 486 233 A2; U.S. PATENT NO. 5 ,030,572). Flax can be transformed by either particle bombardment or *Agrobacterium*-mediated transformation. Recombinase technologies which are useful in practicing the current invention include the *cre-lox*, FLP/FRT and Gin systems. Methods by which these technologies can be used for the purpose described herein are described, for example, in U.S. PATENT NO. 5 ,527,695; Dale And Ow, 1991, Proc. Natl. Acad. Sci. USA 88: 10558-10562; Sauer, 1993, Methods in Enzymology 225: 890-900; Medberry et. al., 1995, Nucleic Acids Res. 23: 485-490. US 5,723,764 describes a method for controlling plant gene expression using *cre*/*lox.*

Selectable marker genes include the neomycin phosphotransferase gene *npt*II (U.S. PATENT NO. 5 ,034,322, U.S. PATENT NO. 5 ,530,196), hygromycin resistance gene (U.S. PATENT NO. 5 ,668,298), *bar* gene encoding resistance to phosphinothricin (U.S. PATENT NO. 5,276,268). EP 0 530 129 A1 describes a positive selection system which enables the transformed plants to outgrow the non-transformed lines by expressing a transgene encoding an enzyme that activates an inactive compound added to the growth media. Useful screenable marker genes include the β-glucuronidase gene (Jefferson et al., 1987, EMBO J. 6: 3901-3907; U.S. PATENT NO. 5 ,268,463) and native or modified green fluorescent protein gene (Cubitt et. al., 1995, Trends Biochem Sci. 20: 448-455; Pang et. al., 1996, Plant Physiol. 112: 893-900). Some of these markers have the added advantage of introducing a trait such as herbicide resistance into the plant of interest providing an additional agronomic value on the input side.

Following transformation by any one of the methods described above, the following procedures can be used to obtain a transformed plant expressing the transgenes of the current invention: select the plant cells that have been transformed on a selective medium; regenerate the plant cells that have been transformed to produce differentiated plants; and select transformed plants expressing the transgene at such that the level of desired polypeptide is obtained in the desired tissue and cellular location.

The examples demonstrate the synthesis of new genetically engineered enzymes for the efficient production of polyhydroxyalkanoate biopolymers in transgenic organisms. In one example, the thiolase and reductase activities encoded by the *phbA* and *phbB* genes have been combined into a single enzyme through the construction of a gene fusion. Use of such a hybrid enzyme and its corresponding gene is advantageous: combining two enzyme activities in a single transcriptional unit reduces the number of genes that need to be expressed in transgenic organisms, and the close proximity of two enzyme activities which catalyse sequential steps in a metabolic pathway. On the fusion enzyme allows for direct transfer of the reaction product from the first catalytic domain to the second domain. These gene fusions can be applied in transgenic microbial or plant crop PHA production systems. The fusions can be expressed in the cytosol or subcellular organelles of higher plants such as the seed of an oil crop (*Brassica,* sunflower, soybean, corn, safflower, flax, palm or coconut), starch accumulating plants (potato, tapioca, cassava), fiber plants (cotton, hemp) or the green tissue of tobacco, alfalfa, switchgrass or other forage crops.

### Examples

The present invention will be further understood by reference to the following examples, which use these general methods and materials:

DNA manipulations were performed on plasmid and chromosomal DNA purified with the Qiagen plasmid preparation or Qiagen chromosomal DNA preparation kits according to manufacturers recommendations. DNA was digested using restriction enzymes (New England Biolabs, Beverly, MA) according to manufacturers recommendations. DNA fragments were isolated from 0.7% agarose-Tris/acetate/EDTA gels using a Qiagen kit. Oligonucleotides were purchased from Biosynthesis or Genesys. DNA sequences were determined by automated sequencing using a Perkin-Elmer ABI 373A sequencing machine. DNA was amplified using the polymerase-chain-reaction in 50 microliter volume using PCR-mix from Gibco-BRL (Gaithersburg, Md) and an Ericomp DNA amplifying machine.

*E. coli* strains were grown in Luria-Bertani medium or 2xYT medium (Sambrook et. al., 1992, in Molecular Cloning, a laboratory manual, 2nd Ed., Cold Spring Harbor Laboratory Press, Cold Spring Harbor, NY). at 37°C, 30 °C or 16°C.

Accumulated PHB was determined by gas chromatographic (GC) analysis, carried out on the lyophilized cell mass. About 20 mg of lyophilized cell mass was subjected to simultaneous extraction and butanolysis at 110°C for 3 hours in 2 mL of a mixture containing (by volume) 90% 1-butanol and 10% concentrated hydrochloric acid, with 2 mg/mL benzoic acid added as an internal standard. The water-soluble components of the resulting mixture were removed by extraction with 3 mL water. The organic phase (1 µL at a split ratio of 1:50 at an overall flow rate of 2 mL/min) was analyzed on an HP 5890 GC with FID detector (Hewlett-Packard Co, Palo Alto, CA) using an SPB-1 fused silica capillary GC column (30 m; 0.32 mm ID; 0.25 µm film; Supelco; Bellefonte, Pa.) with the following temperature profile: 80 °C, 2 min; 10 C° per min to 250 °C; 250 °C, 2 min. Butylbenzoate was used as an internal standard. Molecular weights of the isolated polymers were determined by GPC using a Waters Styragel HT6E column (Millipore Corp., Waters Chromatography Division, Milford, MA) calibrated vs. polystyrene samples of narrow polydispersity. Samples were dissolved in chloroform at 1 mg/mL, 50 µL samples were injected and eluted at 1 mL/min. Detection was performed using a differential refractometer.

Protein samples were denatured by incubation in a boiling water bath (3 minutes) in the presence of 2-mercaptoethanol and sodium dodecylsulphate and subsequently separated on 10%, 15% or 10-20% sodium dodecylsulphate-polyacrylamide gels (SDS-PAGE). After transfer of protein to supported nitrocellulose membranes (Gibco-BRL, Gaithersburg, MD), 3-ketoacyl-CoA thiolase, acetoacetyl-CoA reductase and PHB polymerase were detected using polyclonal antibodies raised against these enzymes in rabbits and horse-radish peroxidase labeled secondary antibodies followed by chemiluminescent detection (USB/Amersham).

β-ketothiolase and NADP-specific acetoacetyl-CoA reductase activities were measured as described by Nishimura et al. (1978, Arch. Microbiol. 116: 21-24) and Saito et al. (1977, Arch. Microbiol. 114: 211-217) respectively. The acetoacetyl-CoA thiolase activity is measured as degradation of a Mg²⁺⁻acetoacetyl-CoA complex by monitoring the decrease in absorbance at 304 nm after addition of cell free extract using a Hewlett-Packer spectrophotometer. The acetoacetyl-CoA reductase activity is measured by monitoring the conversion of NADPH to NADP at 340 nm using a Hewlett-Packer spectrophotometer.

### Example 1: Construction of thiolase-reductase fusion protein (Thredase)

Plasmid pTrc AB11 was constructed using the following techniques essentially as illustrated in Figure 2. The *phbA* gene from *A. eutrophus* was amplified from plasmid pAeT413, a derivative of plasmid pAeT41 (Peoples, O.P. and Sinskey, A.J., 1989, J. Biol. Chem. 264: 15298-15303): by thermal cycling (30 cycles of 40 sec. at 94 °C, 40 sec. at 65 °C and 2 min at 72 °C, followed by a final extension step at 72 °C for 7 min.) with the following primers. The DNA sequence and the amino acid sequence of *phbA* from *A. eutrophus* is shown in SEQ ID NO: 1 and SEQ ID NO: 2
A1F-Kpn contains the ribosome binding site and translational start site; A1F-Bam does not include the translational stop codon. The *A*. *eutrophus phb*B gene was amplified from a derivative of plasmid pAeT41 (Peoples, O.P. and Sinskey, A.J., 1989, J. Biol. Chem. 264: 15298-15303) by thermal cycling (30 cycles of 40 sec. at 94 °C, 40 sec. at 45 °C and 2 min at 72 °C, followed by a final extension step at 72 °C for 7 min.) with the following primers. The DNA sequence and the amino acid sequence of *phbB* from *A. eutrophus* is shown in SEQ ID NO: 5 and SEQ ID NO: 6.

B1 L-Bam contains an ATG initiation codon next to the *BamHI* site but no translational intiation signals; B1L-Xba contains the translational stop codon TGA. The amplified *phbA* gene was then digested with *KpnI* and *BamHI,* and the amplified *phbB* gene was digested with *BamHI* and *XbaI*. Following digestion, the *phbA* gene was cloned into pTrcN which had been digested with *KpnI* and *BamHI* to produce pTrcAF and the *phb*B gene was cloned into *BamHI*/*XbaI*-digested pTrcN to produce pTrcBL.

After confirmation of the DNA sequence of the insert, *phb*B was cloned as a *BamHI*/*XbaI* fragment from pTrcBL into *BamHI*/*XbaI* digested pTrcAF resulting in plasmid pTrcAB11. The resulting hybrid gene encodes for a thiolase-glycine-serine-reductase fusion. The DNA sequence and the amino acid sequence of the AB11 fusion is shown in SEQ ID NO: 9 and SEQ ID NO: 10.

The insertion of the *BamHI* site between *phbA* and *phbB* results in a glycine-serine linker that connects the thiolase and the reductase enzyme and which could be subsequently modified to alter the length and/or sequence of the linker region. Several such derivatives of pTrcAB11 were constructed as follows: pTrcAB11 was digested with *BamHI* and the linearized fragment purified and dephosphorylated with shrimp alkaline phosphatase.

Oligonucleotides were designed to insert the following DNA fragments into the BamHI site. The encoded amino acid sequence is indicated:

Oligonucleotides L5A and L5B (500 pmol) were phosphorylated using T4 polynucleotide kinase and annealed (133 pmol of each primer) and ligated into linearized pTrcAB11. The ligation mixture was electroporated into *E*. *coli* MBX240 and plasmids with the linker inserted between the thiolase and reductase genes were identified by restriction enzyme digestion with BsaWI.

The utility of the fusion constructs was investigated by transforming them into *E*. *coli* MBX240 and examining the integrity of the fusion at the polypeptide level by immunobloting at the protein level by enzyme assays and for the production of PHB. MBX240 was derived from *E. coli* XL1-blue by integration of the *A. eutrophus phaC* gene (Peoples, O.P. and Sinskey, A.J., 1989. J. Biol. Chem. 264: 15298-15303). An altemative approach to the integrated strain would be to have expressed the PHB synthase from a compatible plasmid.

Recombinant strains containing the appropriate fusion plasmid were grown overnight in 2xYT/1% glucose/100 µg/ml ampicillin at 30 C. The grown culture was diluted 1:100 into 50 ml of fresh 2xYT/1% glucose/100 µg/ml ampicillin and incubated at 30 C. Two identical sets of cultures were inoculated, one which was induced with IPTG and one was not induced. Once the culture reached an OD₆₀₀ of 0.6, samples were induced with a final concentration of 1 mM IPTG. Cells were harvested 24 hours after induction by splitting into two 50ml samples and centrifugation at 3000 x g for 10 minutes. Samples of whole cells were retained for analysis of PHB content. The second set of pellets were resuspended in 0.75 ml of lysis buffer (50 mM Tris, 1 mM EDTA, 20% glycerol, pH 8.2) and sonicated (50% output, 2 min. at 50%). The crude extract was then centrifuged (10 min 3000xg, 4 °C) and the supernatant and pellet were separated on 10% SDS-PAGE gels and analyzed by Coomassie staining as well as by immuno-blotting. Immuno-blots were probed with rabbit anti-*A.* eutrophus thiolase and rabbit anti-*A. eutrophus* reductase antibodies. Both antibodies reacted with an Mr = 62kD protein which was absent from the control strain, MBX240 containing the vector pTrcN alone. There was no cross reactivity of the anti-thiolase antibodies with an Mr 42 kD polypeptide or of the reductase antibodies with an Mr 26 kD polypeptide. The soluble protein was then analyzed for thiolase and reductase activity.

The results of these analysis are presented in Table 1 for pTrcAB11 and five derivatives with modified linkers.

**Table 1: Fusion Enzyme Activities**

| **fusion**^{**a**} | **induction**^{**b**} | **thiolase activity**^{**c**} | **reductase activity**^{**c**} | **%PHB**^{**d**} |
|---|---|---|---|---|
| pTrcN | - | 0.03 | 0.05 | 0 |
| | + | 0.03 | 0.03 | 0 |
| AB11 | - | 0.15 | 0.09 | 28.6 |
| | + | 0.32 | 0.07 | 56.3 |
| L5-1 | - | 0.44 | 0.08 | 32.4 |
| | + | 0.97 | 0.12 | 62.5 |
| L5-2 | - | 0.25 | 0.07 | 34.2 |
| | + | 0.37 | 0.09 | 57.6 |
| L5-3 | - | 0.38 | 0.06 | 40.4 |
| | + | 1.18 | 0.09 | 63.6 |
| L5-4 | - | 0.51 | 0.11 | 37.6 |
| | + | 2.21 | 0.17 | 65.3 |
| L5-5 | - | 0.44 | 0.11 | 36.0 |
| | + | 1.85 | 0.23 | 64.1 |

| | | | | |
|---|---|---|---|---|
| ^{a} construct inserted in pTrcN, L5-n indicates an AB11 fusion with a linker derived from the L5 oligonucleotide set; ^{b} culture was induced (+) 1 mM IPTG at an OD600 for 24 hours or was uninduced (-); ^{c} thiolase and reductase activity in U/mg of crude protein extract; ^{d} accumulated PHB as percentage of the cell dry weight. | | | | |

The results presented in Table 1 indicate that these thiolase-reductase fusions have both enzyme activities and result in the production of high levels of PHB.

The fusion encoded by pTrcAB11 was partially purified. A culture of *E. coli* MBX240 (XL1-Blue::*phbC150*) [pTrcAB11] cells grown at 16°C for 33 hours (5.5 g) were resuspended in 11 ml of lysis buffer (50 mM Tris, 1 mM EDTA, 0.05% (w/v) Hecameg, 20% glycerol, pH 8.0) and sonicated (50% output, 2 min at 50%). The crude extract was then centrifuged (10 min 3000xg, 4 °C) and the supernatant was applied to a pre-equilibrated Toyopearl DEAE 650S (Rohm & Haas, PA) column (16.5 x 3.0 cm) in 50 mM NaCl. Unbound protein was washed off with a 50 mM NaCl (300 ml) after which bound protein was eluted with a 50-500 mM NaCl gradient (400 ml total volume). Fractions containing both thiolase and reductase activity (eluted at 250 mM NaCl) were pooled and concentrated/desalted on a 50,000 MW spin column (Amicon). The active protein sample was further purified over a BLUE-SEPHAROSE™ CL6B (Pharmacia Biotech AB, Sweden) column (10.5 cm x 2.6 cm) using the same buffers as for the DEAE but containing different NaCl concentrations. Unbound protein was washed off the column with 250 mM NaCl (200ml) and the remaining protein was eluted in two steps using 750 mM NaCl and 2M NaCl. Two thirds of the thiolase and reductase activities were recovered in the 750 mM NaCl step with the remainder eluting in the 2M NaCl step. Again, fractions containing both thiolase and reductase activity were pooled and concentrated/ desalted on a 50,000 MW spin column. The fusion protein preparation was analyzed by SDS-PAGE proteins detected by either Coomassie Blue staining or Western-blot analysis using anti-β-ketothiolase and anti-acetoacetyl-CoA reductase antibodies. Fractions that contained both β-ketothiolase and acetoacetyl-CoA reductase activity showed a single protein band with an apparent molecular weight of 60 kDa that reacted with both antibodies, confirming both enzyme activities were present on a single polypeptide chain encoded by a single gene.

### Example 2: Construction of reductase-thiolase fusion protein

A hybrid gene that expresses a reductase-glycine-serine-thiolase enzyme was constructed from PCR products containing the reductase and thiolase genes. The following primers
were used to amplify (30 cycles of 40 sec. at 94 °C, 40 sec. at 65 °C and 2 min at 72 °C, followed by a final extension step at 72°C for 7 min.) these genes such that the reductase gene is preceded by a ribosome binding site and does not contain a stop codon. The stop codon of the fusion is provided by the thiolase gene.

The amplified *phbB* gene was digested with *KpnI* and *BamHI,* then cloned into the *KpnI-BamHI* site of pTrcN to produce pTrcBF. The amplified *phbA* gene was digested with *BamHI* and *XbaI,* and was cloned into the *BamHI-XbaI* site of pTrcN to obtain plasmid pTrcAL. The *phb*B gene from pTrcBF was digested with *BamHI-KpnI* and the fragment was inserted it into the *BamHI-KpnI* site of pTrcAL to obtain plasmid pTrcBA, resulting in a fusion gene coding for reductase-glycine-serine-thiolase in one polypeptide. The DNA sequence and the amino acid sequence of the B1A1 fusion is shown in SEQ ID NO: 18 and SEQ ID NO: 19.

### Example 3: Design of PHA synthase-ACP::CoA transferase fusions

The *phaCl* gene encoding PHA synthase 1 of *P. oleovorans* (Huisman et. al., 1991, J. Biol. Chem. 266: 2191-2198) (C3) can be amplified by polymerase chain reaction using the following primers. The DNA sequence and the amino acid sequence of *phbCl* gene of *P. oleovorans* is shown in SEQ ID NO: 20 and SEQ ID NO: 21.

The *pha*G gene encoding acyl-ACP::CoA transferase from *P. putida* (G3) can be amplified by polymerase chain reaction using the following primers. The DNA sequence and the amino acid sequence of *phaG* gene of *P. putida* are shown in SEQ ID NO: 26 and SEQ ID NO: 27.
Fusions of C3 and G3 are subsequently created by cloning either the C3 up and G3 dw PCR products, or the G3 up and C3 dw PCR products as *EcoRI-BamHI* and *BamHI-HindIII* fragments into pTrcN. The resulting plasmids code for either a synthase-transferase fusion (C3G3) or transferase-synthase (G3C3) fusion protein. The DNA sequence and the amino acid sequence of C3G3 is shown in SEQ ID NO: 32 and SEQ ID NO: 33, and the DNA sequence and the amino acid sequence of G3C3 gene are shown in SEQ ID NO: 34 and SEQ ID NO: 35.

### Example 4: Design of PHA synthase-hydratase fusions

The *phaC* gene encoding a PHB synthase fusion from *Z*. *ramigera* (C5) was amplified by polymerase chain reaction using the following primers. The DNA sequence and the amino acid sequence of *phbC* gene of *Z*. *ramigera* are shown in SEQ ID NO: 36 and SEQ ID NO: 37.

The phaJ gene encoding (R)-specific enoyl-CoA transferase from *A*. *caviae* (J 12) can be amplified by polymerase chain reaction using the following primers. The DNA sequence and the amino acid sequence of *phbJ* gene of *A. caviae* are shown in SEQ ID NO: 42 and SEQ ID NO: 43.
Fusions of C5 and J12 are subsequently created by cloning either the C5 up and J12 dw PCR products, or the J12 up and C5 dw PCR products as *EcoRI-BamHI* and *BamHI-HindIII* fragments into pTrcN. The resulting plasmids encode either a synthase-hydratase (C5J12) or hydratase-synthase (J12C5) fusion enzyme. The DNA sequence and the amino acid sequence of C5J12 RE shown in SEQ ID NO: 48 and SEQ ID NO: 49, and the DNA sequence and the amino acid sequence of J12C5 gene are shown in SEQ ID NO: 50 and SEQ ID NO: 51.

### Example 5: Design of broad-substrate range thiolase-reductase fusions

The *bktB* gene encoding thiolase II of *R. eutropha* (Slater et al. J. Bacteriol. (1998) 180, 1979-1987) (A1-11) can be amplified by polymerase chain reaction using the following primers. The DNA sequence and the amino acid sequence of *bktB* gene of *R. eutropha* are shown in SEQ ID NO: 52 and SEQ ID NO: 53.

The *phaB* gene encoding acyl-CoA reductase from *R. eutropha* (B 1) is amplified by polymerase chain reaction using the primers described in Example 1. Fusions of AI-II and B1 are subsequently created by cloning either the A1-II up and B1 dw PCR products, or the B1 up and A1-II dw PCR products as *EcoRI-BamHI* and *BamHI-HindIII* fragments into pTrcN. The resulting plasmids encode either a thiolase-reductase (A1-IIB1) or reductase-thiolase (B1A1-II)) fusion enzyme. The DNA sequence and the amino acid sequence of A1-IIB1 is shown in SEQ ID NO: 58 and SEQ ID NO: 59, and the DNA sequence and the amino acid sequence of B1A1-II gene are shown in SEQ ID NO: 60 and SEQ ID NO: 61.

Modifications and variations of the present invention will be obvious to those of skill in the art from the foregoing detailed description.

## Claims

1. A bifunctional fusion protein having a formula selected from the group consisting of E1-Lₙ-E2 and E2-Lₙ-E1.
wherein E1 and E2 catalyze successive reactions in a polyhydroxyalkanoate biosynthetic pathway and are each selected from the group consisting of β-ketothiolases, acyl-CoA reductases, polyhydroxyalkanoate synthases, polyhydroxybutyrate synthases, phasins, enoyl-CoA hydratases, and beta-hydroxyacyl-ACP::coenzyme-A transferase, in which linker Lₙ is a peptide of n amino acids that link E1 to E2 or E2 to E1, and wherein the bifunctional fusion protein possesses the enzymatic activities of E1 and E2.

2. The fusion protein of claim 1 wherein E1 and E2 are selected from the group consisting of beta-ketothiolase (phbA) and acyl-CoA reductase (phbB); phbB and phbA; PHA synthase (phaC) and phasin (phaP); phaP and phaC; phaC and beta-hydroxyacyl-ACP::coenzyme A transferase (phbG); phbG and phaC; phaC and enoyl-CoA hydratases (phaJ); and phaJ and phaC.

3. The fusion protein of claim 1 wherein n in the linker is between zero and 50 amino acids.

4. The fusion protein of claim 1 wherein the linker is glycine-serine.

5. The fusion protein of claim 1 expressed in a plant.

6. The fusion protein of claim 1 expressed in a bacterium.

7. A polynucleotide encoding a bifunctional fusion protein as defined by any one of Claims 1 to 4.

8. The polynucleotide of claim 7 comprising a promoter for expression in plants.

9. The polynucleotide of claim 8 comprising a promoter specific for expression in a tissue, plastid, or other organ.

10. The polynucleotide of claim 8 comprising a regulatable promoter.

11. A plant comprising a polynucleotide according to any one of claims 7-10.

12. A plant that expresses a bifunctional fusion protein according to any one of claims 1 to 4.

13. A bacterium comprising a polynucleotide according to any one of claims 7-10.

14. A bacterium that expresses a bifunctional fusion protein according to any one of claims 1 to 4.

## Patentansprüche

1. Bifunktionelles Fusionsprotein mit einer Formel, die aus der Gruppe ausgewählt ist, die aus E1-Lₙ-E2 und E2-Lₙ-E1 besteht,
wobei E1 und E2 aufeinanderfolgende Reaktionen in einem Biosyntheseweg für Polyhydroxyalkanoate kalalysieren und beide aus der Gruppe ausgewählt sind, die besteht aus β-Ketothiolasen, Acyl-CoA-Reduktasen, Polyhydroxyalkanoat-synthasen, Polyhydroxybutyratsynthasen, Phaslnen, Enoyl-CoA-Hydratasen und Beta-Hydroxyacyl-ACP:Coenzym-A-Transferase, wobei der Linker Lₙ ein Peptid aus n Aminosäuren ist, die E1 mit E2 oder E2 mit E1 verknüpfen, und wobei das bifunktionelle Fusionsprotein die Enzymaktivltäten von E1 und E2 besitzt.

2. Fusionsprotein nach Anspruch 1, wobei E1 und E2 aus der Gruppe ausgewählt sind, die besteht aus β-Ketothiolase (phbA) und Acyl-CoA-Reduktase (phhB), phbB und phbA, PHA-Synthase (phaC) und Phasin (phaP), phaP und phaC, phaC und Beta-Hydroxyacyl-ACP::Coenzym-A-Transferase (phbG), phbG und phac, phaC und Enoyl-CoA-Hydratasen (phaJ) sowie phaJ und phaC.

3. Fusionsprotein nach Anspruch 1, wobei n im Linker zwischen null und 50 Aminosäuren liegt.

4. Fusionsprotein nach Anspruch 1, wobei der Linker Glycin-Serin ist.

5. Fusionsprotein nach Anspruch 1, exprimiert in einer Pflanze.

6. Fusionsprotein nach Anspruch 1, exprimiert in einem Bakterium.

7. Polynucleotid, das für ein bifunktionelles Fusionsprotein, wie es durch einen beliebigen der Ansprüche 1 bis 4 definiert ist, codiert.

8. Polynucleotid nach Anspruch 7, das einen Promotor für die Expression in Pflanzen umfasst.

9. Polynucleotid nach Anspruch 8, das einen für die Expression in einem Gewebe, einer Plastide oder einem anderen Organ spezifischen Promotor umfasst

10. Polynucleotid nach Anspruch 8, das einen regulierbaren Promotor umfasst.

11. Pflanze, die ein Polynucleotid gemäß einem beliebigen der Ansprüche 7-10 umfasst.

12. Pflanze, die ein bifunktionelles, Fusionsprotein gemäß einem beliebigen der Ansprüche 1 bis 4 exprimiert.

13. Bakterium, das ein Polynucleotid gemäß einem beliebigen der Ansprüche 7-10 umfasst.

14. Bakterium, das ein bifunktionelles Fusionsprotein gemäß einem beliebigen der Ansprüche 1 bis 4 exprimiert.

## Revendications

1. Protéine de fusion bifonctionnelle ayant une formule choisie dans le groupe constitué par E1-Lₙ-E2 et E2-Lₙ-E1,
dans laquelle E1 et E2 catalysent des réactions successives dans une voie de biosynthèse de polyhydroxyalcanoate et sont chacun choisis dans le groupe constitué par les β-cétothiolases, les acyl-CoA réductases, les polyhydroxyalcanoate synthases, les polyhydroxybutyrate synthases, les phasines, les énoyl-CoA hydratases, et la beta-hydroxyacyl-ACP::coenzyme-A transférase, dans laquelle le lieur Lₙ est un peptide de n acides aminés qui lient E1 à E2 ou E2 à E1, et dans laquelle la protéine de fusion bifonctionnelle possède les activités enzymatiques de E1 et de E2.

2. Protéine de fusion selon la revendication 1, dans laquelle E1 et E2 sont choisis dans le groupe constitué par la bêta-cétothiolase (phbA) et l'acyl-CoA réductase (phbB) ; phbB et phbA ; la PHA synthase (phaC) et la phasine (phaP) ; phaP et phaC ; phaC et la beta-hydroxyacyl-ACP::coenzyme-A transférase (phbG) ; phbG et phaC ; phaC et les énoyl-CoA hydratases (phaJ) ; et phaJ et phaC.

3. Protéine de fusion selon la revendication 1, dans laquelle.n dans le lieur est compris entre 0 et 50 acides aminés.

4. Protéine de fusion selon la revendication 1, dans laquelle le lieur est la glycine-sérine.

5. Protéine de fusion selon la revendication 1 exprimée dans une plante.

6. Protéine de fusion selon la revendication 1 exprimée dans une bactérie.

7. Polynucléotide codant pour une protéine de fusion bifonctionnelle telle que définie par l'une quelconque des revendications 1 à 4.

8. Polynucléotide selon la revendication 7, comprenant un promoteur pour l'expression dans des plantes.

9. Polynucléotide selon la revendication 8, comprenant un promoteur spécifique pour l'expression dans un tissu, un plaste, ou autre organe.

10. Polynucléotide selon la revendication 8, comprenant un promoteur modulable.

11. Plante comprenant un polynucléotide selon l'une quelconque des revendications 7 à 10.

12. Plante qui exprime une protéine de fusion bifonctionnelle selon l'une quelconque des revendications 1 à 4.

13. Bactérie comprenant un polynucléotide selon l'une quelconque des revendications 7 à 10.

14. Bactérie qui exprime une protéine de fusion bifonctionnelle selon l'une quelconque des revendications 1 à 4.
